# EUROPÄISCHE PATENTSCHRIFT

(11) **EP 1 647 270 B1**
(45) Veröffentlichungstag und Bekanntmachung des Hinweises auf die Patenterteilung: **17.06.2009**
(21) Anmeldenummer: 05027658.3
(22) Anmeldetag: 19.03.1999
(51) Int. Cl.: A61K 9/50, B01J 13/02

(54) **Verfahren zum Aufbringen mehrerer Schichten von Beschichtungssubstanzen auf Templatpartikel**
Process for applying several layers of coating substances to template particles
Méthode pour l'application de plusieurs couches de substances de revêtement sur une matrice

(30) Priorität: 19.03.1998 DE 19812083; 15.07.1998 EP 98113181; 22.02.1999 DE 19907552
(43) Veröffentlichungstag der Anmeldung: 19.04.2006
(62) Teilanmeldung aus: 99911804.5
(73) Patentinhaber: Max-Planck-Gesellschaft zur Förderung der Wissenschaften e.V., 80539 München (DE)
(72) Erfinder: Donath, Edwin, Prof. Dr., 04316 Leipzig (DE); Sukhorukov, Gleb, Dr., Moscow 115280 (RU); Lerche, Karl-Heinz, Dr., 10243 Berlin (DE); Voigt, Andreas, Dr., 12621 Berlin (DE); Bäumler, Hans, Dr., 12437 Berlin (DE); Caruso, Frank, Prof. Dr., Preston Victoria 3072 (AU); Möhwald, Helmuth, Prof. Dr., 55411 Bingen (DE)
(74) Vertreter: Dey, Michael

(56) Entgegenhaltungen:
- DE-A1- 4 312 970
- GB-A- 2 135 954
- GB-A- 2 145 992
- US-A- 3 429 827
- CARUSO F ET AL: "INFLUENCE OF POLYELECTROLYTE MULTILAYER COATINGS ON FOERSTER RESONANCE ENERGY TRANSFER BETWEEN 6-CARBOXYFLUORESCEIN AND RHODAMINE B-LABELED PARTICLES IN AQUEOUS SOLUTION" JOURNAL OF PHYSICAL CHEMISTRY. B, MATERIALS, SURFACES, INTERFACES AND BIOPHYSICAL, WASHINGTON, DC, US, Bd. 102, 24. Februar 1998 (1998-02-24), Seiten 2011-2016, XP000852731 ISSN: 1089-5647

## Beschreibung

Die Erfindung betrifft ein Verfahren zum Aufbringen mehrerer Schichten von Beschichtungssubstanzen auf Templatpartikel, um Nano- bzw. Mikrokapseln herzustellen.

Mikrokapseln sind in verschiedenen Ausführungsformen bekannt und werden insbesondere für die kontrollierte Freisetzung und den zielgerichteten Transport von pharmazeutischen Wirkstoffen sowie zum Schutz von empfindlichen Wirkstoffen, wie etwa Enzymen und Proteinen verwendet (siehe z.B. D.D.Lewis, "Biodegradable Polymers and Drug Delivery Systems", M.Chasin and R.Langer, Hrsg. (Marcel Decker, New York, 1990); J.P.McGee et al., J.Control.Release 34 (1995), 77).

Mikrokapseln können durch mechanisch-physikalische Verfahren, wie z.B. Versprühen und nachfolgende Beschichtung hergestellt werden. Die auf diese Weise erhältlichen Mikrokapseln weisen jedoch eine Reihe von Nachteilen auf. Insbesondere ist es mit den bekannten mechanisch-physikalischen Verfahren nicht möglich, Mikrokapseln mit einer Größe von < 10 µm (Durchmesser) herzustellen. Vielmehr sind nur Mikrokapseln mit relativ großen Durchmessern erhältlich, deren Anwendungsbereich jedoch aufgrund ihrer Größe beschränkt ist. Weiterhin wird bei den bekannten mechanisch-physikalischen Verfahren keine monodisperse Kapselverteilung, sondern vielmehr eine uneinheitliche Verteilung von Kapseln verschiedener Größe erhalten. Auch dies ist für viele Anwendungen, bei denen die Größe der Kapsel wichtig ist, nachteilig.

Neben den mechanisch-physikalischen Verfahren sind auch chemische Verfahren zur Herstellung von Mikrokapseln bekannt. So können Mikrokapseln durch Grenzflächenpolymerisation bzw. -kondensation oder durch Polymerphasentrennung aus einem Polymer/Lösungsmittelgemisch hergestellt werden (B.Miksa et al., Colloid Polym.Sci.273 (1995), 47; G.Crotts et al., J.Control.Release 35 (1995), 91; S.L.Regen et al., J.Am.Chem.Soc.106 (1984), 5756). Aber auch die durch bekannte chemische Verfahren hergestellten Mikrokapseln weisen eine Reihe von Nachteilen auf. Insbesondere sind eine hohe Polydispersität, eine ungleichmäßige Umhüllung sowie oftmals eine Verfestigung des Kerns zu beobachten. Ein weiterer wesentlicher Nachteil der bekannten chemischen Verfahren liegt in der Verwendung von organischen Lösungsmitteln und polymerisierbaren organischen Monomeren, was zu beträchtlichen Beschränkungen hinsichtlich der verwendbaren einzukapselnden Wirkstoffe führt. Insbesondere die oftmals dadurch notwendige Verwendung von mit Wasser nicht mischbaren organischen Flüssigkeiten als Kernmaterial schränkt den Anwendungsbereich solcher Mikrokapseln, gerade im Hinblick auf Proteine oder Enzyme drastisch ein.

Ein weiteres System, das zur Einkapselung von anorganischen und organischen Materialien verwendet wurde, sind Lipidliposomen (D.D.Lasic, "Liposomes: From Physics to Applications" (Elsevier, Amsterdam, 1993); S.L.Regen et al., J.Am.Chem.Soc.106 (1984), 2446). Die Einkapselung von Wirkstoffen in Lipidliposomen gestattet die Herstellung von Mikrokapseln unter relativ schonenden Bedingungen, weshalb Liposomen als Trägersysteme für verschiedene pharmazeutische und kosmetische Wirkstoffe verwendet werden. Die biologische, chemische und mechanische Stabilität solcher Liposomkapseln ist jedoch sehr gering, was die allgemeine Verwendbarkeit solcher Kapseln limitiert. Einen weiteren Nachteil stellt die geringe Permeabilität von Liposomenkapseln, insbesondere für polare Moleküle, dar, die einen Stoffaustausch mit dem Umgebungsmedium verhindert.
Bei einem weiteren Verfahren zur Herstellung von Mikrokapseln werden zunächst Mischungen aus dem einzuschließenden Material und einem mit z.B. Ca²+-lonen verfestigbaren Polyelektrolytbestandteil gebildet. Diese Mischung wird in Form kleinster Tröpfchen in ein Ca²⁺-Bad eingebracht, wobei sich eine Gelstruktur bildet, die dann in weiteren Verfahrensschritten mit einer Polyelektrolytkapsel umgeben werden kann. Eine Weiterentwicklung solcher Verfahren wird in DE 33 06 259 A1 beschrieben, wobei auf die Verwendung von Ca²⁺ verzichtet werden kann. Der hauptsächliche Nachteil dieser Verfahren besteht darin, daß die Größe der herstellbaren Mikrokapseln nach unten auf etwa 50 µm (Durchmesser) begrenzt ist und die Wandstärke der erhaltenen Mikrokapseln mindestens 100 nm beträgt.

In DE-A-40 26 978 ist ein Verfahren zur Beschichtung von flächigen Trägern beschrieben, wobei ein Träger so modifiziert wird, daß er flächenweit Ionen oder ionisierbare Verbindungen mit gleichsinniger Ladung trägt und eine oder mehrere Schichten aus organischen Materialien, die in jeder Schicht gleichsinnig geladene Ionen enthalten, aus einer Lösung solcher organischer Materialien auf den modifizierten Träger aufgetragen wird, wobei das organische Material für die erste Schicht Ionen mit entgegengesetztem Ladungsinn zum Ladungssinn der lonenmodifizierung des Trägers aufweist und im Falle von mehreren Schichten abwechselnd weitere Schichten mit Ionen mit dem jeweils entgegengesetzten Ladungssinn zur vorhergehenden in gleicher Weise wie die erste Schicht aufgetragen werden. Als Träger werden anorganische oder organische Trägermaterialien mit ebenmäßiger Oberfläche offenbart. Es findet sich keinerlei Hinweis auf die Verwendung von Mikropartikeln als Trägermaterialien oder auf eine Auflösung der Trägermaterialien nach der Beschichtung.

Eine Aufgabe der Erfindung bestand deshalb darin, Kapseln mit einem geringen Durchmesser bereitzustellen, in denen Materialen, wie etwa Makromoleküle, Präzipitate, Flüssigkeiten oder Gase, eingeschlossen werden können. Die Kapseln sollten weiterhin eine hohe Stabilität und Hüllen mit geringer Wandstärke aufweisen, die insbesondere für Ionen und kleine Moleküle durchlässig sind.

Die Aufgabe wird erfindungsgemäß gelöst durch das Aufbringen mehrerer aufeinanderfolgender Schichten auf einen Träger durch ein Filtrationsverfahren. Durch diese Methode können mit Polyelektrolytmoleküle beschichtete Kapseln effizient, auf einfache Weise und in großem Maßstab hergestellt werden. Überraschenderweise können selbst empfindliche Templatpartikel wie biologische Zellen durch ein Filtrationsverfahren beschichtet werden.

Die Erfindung betrifft somit ein Verfahren zum Aufbringen mehrerer Schichten von Beschichtungssubstanzen auf Templatpartikel umfassend die Schritte:
(a) Inkontraktbringen des Templatpartikels mit einer ersten Beschichtungssubstanz in einem fluiden, vorzugsweise wässrigen Reaktionsmedium in einem Reaktionsraum, der auf mindestens einer Seite durch eine Filtrationsmembran begrenzt ist, unter Bedingungen, bei denen eine Schicht der ersten Beschichtungssubstanz auf dem Templatpartikel gebildet wird,
(b) Ableiten von mindestens einem Teil des Reaktionsmediums mit gegebenenfalls darin enthaltener überschüssiger erster Beschichtungssubstanz durch die Filtrationsmembran in einen Filtratraum, wobei vorzugsweise die überschüssige erste Beschichtungssubstanz im wesentlichen vollständig abgeleitet wird,
(c) Inkontaktbringen des Templatpartikels mit einer zweiten Beschichtungssubstanz in einem fluiden Reaktionsmedium in einem Reaktionsraum der auf mindestens einer Seite durch eine Filtrationsmembran begrenzt ist, unter Bedingungen, bei denen eine Schicht der zweiten Beschichtungssubstanz auf dem Templatpartikel gebildet wird,
(d) Ableiten von mindestens einem Teil des Reaktionsmediums mit gegebenenfalls darin enthaltener überschüssiger zweiter Beschichtungssubstanz durch die Filtrationsmembran in einen Filtratraum, wobei vorzugsweise die überschüssige zweite Beschichtungssubstanz vorzugsweise im wesentlichen vollständig abgeleitet wird, und
(e) gegebenenfalls mehrmaliges Wiederholen der Schritte (a) und (b) oder/und (c) und (d).

Als erste und zweite Beschichtungssubstanzen werden vorzugsweise jeweils entgegengesetzt geladene Polyelektrolytspezies oder Gemische von Polyelektrolyspezies verwendet. Weiterhin können als Beschichtungssubstanzen auch Nanopartikel, amphiphile Polyelektrolyte, Lipide oder/und Tenside verwendet werden.

Unter Polyelektrolyten werden allgemein Polymere mit ionisch dissoziierbaren Gruppen, die Bestandteil oder Substituent der Polymerkette sein können, verstanden. Üblicherweise ist die Zahl dieser ionisch dissoziierbaren Gruppen in Polyelektrolyten so groß, daß die Polymeren in der dissoziierten Form (auch Polyionen genannt) wasserlöslich sind. Hierin werden unter dem Begriff Polyelektrolyte auch lonomere verstanden, bei denen die Konzentration der ionischen Gruppen für eine Wasserlöslichkeit nicht ausreichend sind, die jedoch genügend Ladungen aufweisen, um eine Selbstassemblierung einzugehen. Bevorzugt umfasst die Hülle "echte" Polyelektrolyte. Je nach Art der dissoziierbaren Gruppen werden Polyelektrolyte in Polysäuren und Polybasen unterteilt. Aus Polysäuren entstehen bei der Dissoziation unter Abspaltung von Protonen Polyanionen, die sowohl anorganische als auch organische Polymere sein können. Beispiele für Polysäuren sind Polyphosphorsäure, Polyvinylschwefelsäure, Polyvinyl-sulfonsäure, Polyvinylphosphonsäure und Polyacrylsäure. Beispiele für die entsprechenden Salze, die auch als Polysalze bezeichnet werden, sind Polyphosphat, Polysulfat, Polysulfonat, Polyphosphonat und Polyacrylat.

Polybasen enthalten Gruppen, die in der Lage sind, Protonen, z.B. durch Reaktion mit Säuren unter Salzbildung, aufzunehmen. Beispiele für Polybasen mit ketten - bzw. seitenständigen dissoziierbaren Gruppen sind Polyethylenimin, Polyvinylamin und Polyvinylpyridin. Polybasen bilden durch Aufnahme von Protonen Polykationen.

Erfindungsgemäß geeignete Polyelektrolyte sind sowohl Biopolymere, wie etwa Alginsäure, Gummi arabicum, Nucleinsäuren, Pektine, Proteine und andere, sowie chemisch modifizierte Biopolymere, wie etwa ionische oder ionisierbare Polysaccharide, z.B. Carboxymethylcellulose, Chitosan und Chitosansulfat, Ligninsulfonate sowie synthetische Polymere, wie etwa Polymethacrylsäure, Polyvinylsulfonsäure, Polyvinylphosphonsäure und Polyethylenimin.

Es können lineare oder verzweigte Polyelektrolyte eingesetzt werden. Die Verwendung verzweigter Polyelektrolyte führt zu weniger kompakten Polyelektrolytmultifilmen mit einem höheren Grad der Wandporosität. Zur Erhöhung der Kapselstabilität können Polyelektrolytmoleküle innerhalb oder/und zwischen den einzelnen Schichten vernetzt werden, z.B. durch Crosslinking von Aminogruppen mit Aldehyden. Weiterhin können amphiphile Polyelektrolyte, z.B. amphiphile Block- oder Randomcopolymere mit partiellem Polyelektrolytcharakter zur Verringering der Permeabilität gegenüber polaren kleinen Molekülen eingesetzt werden. Solche amphiphilen Copolymere bestehen aus Einheiten unterschiedlicher Funktionalität, z.B. einerseits sauren oder basischen Einheiten und andererseits hydrophoben Einheiten wie Styrolen, Dienen oder Siloxanen etc. die als Blöcke oder statistisch verteilt über das Polymer angeordnet sein können. Durch Verwendung von Copolymeren, die als Funktion äußerer Bedingungen ihre Struktur ändern, können die Kapselwände bezüglich ihrer Permeabilität oder anderer Eigenschaften definiert gesteuert werden. Hierzu bieten sich beispielsweise Copolymere mit einem Poly(N-isopropyl-acrylamid)-Anteil, z.B. Poly(N-isopropylacrylamid-acrylsäure) an, die über das Gleichgewicht von Wasserstoffbrückenbindungen ihre Wasserlöslichkeit als Funktion der Temperatur ändern, was mit einer Quellung einhergeht.
Durch Verwendung von unter bestimmten Bedingungen abbaubaren, z.B. photo-, säure-, base- oder salzlabilen Polyelektrolyten kann über die Auflösung der Kapselwände die Freisetzung von eingeschlossenen Wirkstoffen gesteuert werden. Weiterhin können für bestimmte Anwendungsmöglichkeiten auch leitende Polyelektrolyten oder Polyelektrolyten mit optisch aktiven Gruppen als Kapselkomponenten verwendet werden.

Durch geeignete Wahl der Polyelektrolyte ist es möglich, die Eigenschaften und Zusammensetzung der Polyelektrolythülle der erfindungsgemäßen Kapseln definiert einzustellen. Insbesondere bei schichtweise aufgebauten Polyelektrolythüllen kann die Zusammensetzung der Hüllen durch die Wahl der Substanzen beim Schichtaufbau in weiten Grenzen variiert werden. Grundsätzlich ergeben sich keine Einschränkungen hinsichtlich der zu verwendenden Polyelektrolyte bzw. lonomere, solange die verwendeten Moleküle eine genügend hohe Ladung aufweisen oder/und die Fähigkeit besitzen, über andere Wechselwirkungsarten, wie beispielsweise Wasserstoffbrückenbindungen und/oder hydrophobe Wechselwirkungen, eine Bindung mit der darunter liegenden Schicht einzugehen.

Geeignete Polyelektrolyte sind somit sowohl niedermolekulare Polyelektrolyte bzw. Polyionen als auch makromolekulare Polyelektrolyte, beispielsweise Polyelektrolyte biologischer Herkunft.

Von besonderer Bedeutung für die Verwendung der Kapseln ist die Permeabilität der Hüllwand. Wie bereits oben ausgeführt, ermöglicht die Vielzahl der zur Verfügung stehenden Polyelektrolyte die Herstellung einer Vielzahl von Hüllkompositionen mit unterschiedlichen Eigenschaften. Insbesondere kann die elektrische Ladung der Außenhülle dem Anwendungszweck angepaßt werden. Zudem kann die Innenhülle an jeweils verkapselte Wirkstoffe angepaßt werden, wodurch z.B. eine Stabilisierung des Wirkstoffs erzielt werden kann. Daneben kann auch die Permeabilität der Hüllwand durch die Wahl der Polyelektrolyte in der Hülle und durch die Wanddicke sowie die Umgebungsbedingungen beeinflußt werden. Dadurch ist eine selektive Gestaltung der Permeabilistätseigenschaften sowie eine definierte Veränderung dieser Eigenschaften möglich.

Die Permeabilitätseigenschaften der Hülle können durch Poren in mindestens einer der Polyelektrolytschichten weiter modifiziert werden. Solche Poren können bei geeigneter Wahl durch die Polyelektrolyten selbst gebildet werden. Neben den Polyelektrolyten kann die Hülle aber auch andere Substanzen umfassen, um eine gewünschte Permeabilität zu erzielen. So kann insbesondere durch Einbringen von Nanopartikeln mit anionischen oder/und kationischen Gruppen oder von grenzflächenaktiven Substanzen, wie etwa Tensiden oder/und Lipiden, die Permeabilität für polare Komponenten gesenkt werden. Durch die inkorporation von selektiven Transportsystemen, wie z.B. Carriern oder Kanälen, in die Polyelektrolythülle, insbesondere in Lipidschichten, ist eine genaue Anpassung der transversalen Transporteigenschaften der Hülle an den jeweiligen Anwendungszweck möglich. Die Poren oder Kanäle der Hüllwand können durch chemische Modifizierung oder/und Änderung der Umgebungsbedingungen gezielt geöffnet bzw. verschlossen werden. So führt beispielsweise eine hohe Salzkonzentration des Umgebungsmediums zu einer hohen Durchlässigkeit der Hüllwand.

Eine besonders bevorzugte Modifizierung der Permeabilität von Polyelektrolythüllen kann durch Abscheidung von Lipidschichten oder/und amphiphiler Polyelektrolyten auf der Polyelektrolythülle nach Desintegration der Templatpartikel erreicht werden. Auf diese Weise kann die Permeabilität der Polyelektrolythüllen für kleine und polare Moleküle sehr stark vermindert werden. Beispiele für Lipide, die auf den Polyelektrolythüllen abgeschieden werden können, sind Lipide, die mindestens eine ionische oder ionisierbare Gruppe tragen, z.B. Phospholipide wie etwa Dipalmitoylphosphatidinsäure oder zwitterionische Phospholipide wie etwa Dipalmitoylphosphatidylcholin oder auch Fettsäuren bzw. entsprechende langkettige Alkylsulfonsäuren. Bei Verwendung zwitterionischer Lipide können Lipidmultischichten auf der Polyelektrolythülle abgeschieden werden. Auf den Lipidschichten können anschließend weitere Polyelektrolytschichten abgeschieden werden.

Als Templatpartikel können sowohl anorganische Materialien, z.B. Metalle, Keramiken, Oxide oder Salzkristalle als auch organische Materialien wie Polymerlatizes oder Melaminformaldehydpartikel, Lipidvesikel oder biologische Templatpartikel eingesetzt werden. Ebenfalls geeignet sind Emulsionströpfchen. Die Größe der Templatpartikel kann - insbesondere bei Verwendung biologischer Templatmaterialien - bis zu 50 µm erreichen. In den meisten Fällen ist die Größe der Templatpartikel jedoch bis zu 10 µm, besonders bevorzugt 5 nm bis 5 µm. Die Form der Templatpartikel ist nicht kritisch. Sowohl sphärische als auch anisotrope Partikel können beschichtet werden.

Es können auch Aggregate von Subpartikeln als Ausgangskerne (Templatpartikel) zur Beschichtung mit Polyelektrolyten eingesetzt werden. Diese Aggregate können gegebenenfalls in vorgeformtem oder präformiertem Zustand eingesetzt werden. Eine solche Präformierung kann beispielsweise durch Anlegen externer elektrischer Gleichstrom- oder/und Wechselfelder bzw. Magnetfelder auf Suspensionen mit Subpartikeln erzielt werden. Durch vorgeformte Aggregate kann die Form der Kapseln bestimmt werden. Weiterhin können solche Aggregate mit einer hohen Einheitlichkeit hinsichtlich der Größenverteilung erhalten werden (Monodispersität). Ebenso geeignet sind jedoch auch nichtvorgeformte Aggregate. Von besonderem Interesse sind Aggregate in sphärischer Form.

Die verwendeten Templatpartikel müssen nicht notwendigerweise geladen sein, um eine Selbstassemblierung von Polyelektrolytschichten zu ermöglichen. Vielmehr kann auf nichtgeladene Kerne ein geladener Precursorfilm aufgebracht werden, der an die Templatpartikel durch andere Wechselwirkungen, beispielsweise hydrophobe Wechselwirkungen, gebunden ist.

Nach Aufbringen der gewünschten Anzahl von Polyelektrolytschichten können die umhüllten Templatpartikel - sofern gewünscht - desintegriert, insbesondere zerkleinert oder aufgelöst werden. Dabei verbleiben "leere" Kapseln mit einer Polyelektrolythülle. Die Auflösung der Templatpartikel wird unter Bedingungen durchgeführt, bei denen die Hüllen intakt bleiben. Eine Auflösung kann je nach Wahl des Templatpartikelmaterials z.B. thermisch oder chemisch erfolgen. Die bei der Auflösung entstehenden niedermolekularen Kernbestandteile können durch die Poren der Hülle nach außen gelangen. Auf diese Weise erhält man Kapseln mit Polyelektrolythüllen, die einen "leeren" Kern enthalten. Auf die leeren Polyelektrolytmolekülen können anderen Beschichtungssubstanzen aufgebracht werden.

Nach Desintegration der Templatpartikel kann im Inneren der Kapselhülle eine Flüssigphase vorliegen. Grundsätzlich können die Kapseln jede Flüssigkeit in ihrem Innern enthalten. z.B. eine wäßrige Flüssigkeit, insbesondere eine wäßrige Salzlösung oder Wasser, oder auch organische Lösungsmittel, insbesondere mit Wasser nicht mischbare Lösungsmittel wie Alkohole oder Kohlenwasserstoffe mit mindestens 4 C-Atomen. Weiterhin können die Kapseln in ihrem Inneren auch Feststoffe oder Gase enthalten.
Bevorzugt werden teilvernetzte Melaminformaldehydpartikel als auflösbare Templatpartikel eingesetzt, die durch Einstellung des pH-Werts in dem die umhüllten Partikel enthaltenden Medium auf einen sauren Wert, z.B. ≤ 1,5, aufgelöst werden können, während die Hüllschicht selbst intakt bleibt. Die teilvernetzten Melaminformaldehydpartikel können auch durch chemische Reaktionen, insbesondere durch Sulfonierung in wäßrigen Medien aufgelöst werden. Als Sulfonierungsagentien werden bevorzugt Alkalisulfate, Alkalihydrogensulfite und andere wasserlösliche Salze der schwefeligen Säure verwendet. Weitere Beispiele für auflösbare Templatpartikel sind lösliche Polymerkerne, z.B. Harnstoff-Formaldehydpartikel, oder Salzkristalle.

Außerdem können als Templatmaterialien beispielsweise Zellen, z.B. eukaryontische Zellen, wie etwa Säugererythrozyten oder Pflanzenzellen, einzellige Organismen wie etwa Hefen, Bakterienzellen wie etwa E.coli Zellen, Zellaggregate, subzelluläre Partikel wie etwa Zellorganelle, Pollen, Membranpräparationen oder Zellkerne, Viruspartikel und Aggregate von Biomolekülen, z.B. Proteinaggregate wie etwa Immunkomplexe, kondensierte Nukleinsäuren, Ligand-Rezeptor-Komplexe etc. verwendet werden. Das erfindungsgemäße Verfahren eignet sich auch zur Verkapselung lebender biologischer Zellen und Organismen. Ebenso als Template geeignet sind Aggregate amphiphiler Materialien, insbesonder Membranstrukturen wie etwa Vesikel, z.B. Liposomen oder Micellen sowie andere Lipidaggregate.

Die Desintegration biologischer Templatpartikel kann durch Zugabe von Lysereagenzien erfolgen. Dabei sind Lysereagenzien geeignet, die biologische Materialien wie Proteine oder/und Lipide auflösen können. Vorzugsweise enthalten die Lysereagenzien ein Deproteinisierungsmittel, beispielsweise Peroxoverbindungen wie etwa H₂O₂ oder/und Hypochloritverbindungen wie etwa Natrium- oder Kaliumhypochlorit. Überraschenderweise erfolgt die Desintegration der Templatpartikel innerhalb einer kurzen Inkubationsdauer, z.B. 1 min bis 1 h bei Raumtemperatur. Die Desintegration der Templatpartikel ist weitgehend vollständig, da selbst bei elektronenmikroskopischer Betrachtung der verbleibenden Hüllen keine Reste der Partikel mehr nachweisbar sind. Bei Einbau biologischer Polyelektrolyte in die Hülle können leere Schichten auch innerhalb der Polyelektrolythülle erzeugt werden.

Die bei der Desintegration der Templatpartikel gebildeten Fragmente, z.B. im Fall von teilvernetzten Melaminformaledhypartikeln, die bei Auflösung entstandenen Oligomere, können durch Poren, insbesondere Nanoporen, der Hüllwand aus dem Inneren der Kapseln nach außen austreten. Anschließend können sie - sofern gewünscht - von den Kapseln abgetrennt werden. Diese Abtrennung kann durch dem Fachmann bekannte Verfahren durchgeführt werden, z.B. durch Dialyse, Filtration oder/und Zentrifugation abgetrennt. Eine Abtrennung von Templatpartikelfragmenten ist oftmals jedoch nicht notwendig. Die Kapseln können auch ohne Abtrennungsschritt verwendet werden.

Die Templatpartikel werden vorzugsweise aus Partikeln mit einem Durchmesser von bis zu 50 µm, insbesondere bis zu 10 µm ausgewählt. Vorzugsweise werden auflösbare Partikel wie zuvor genannt, z.B. teilvernetzte Melaminformaldehydpartikel, biologische Partikel oder Aggregate biologischer oder/und amphiphiler Materialien, insbesondere biologische Aggregate wie Zellen, Zellaggregate, Viruspartikel etc. verwendet.

Um eine vollständige Entfernung überflüssiger Beschichtungssubstanz nach einem Beschichtungsschritt zu ermöglichen, wird während oder/und nach Schritt (b) oder/und (d) ein Waschmedium, z.B. Wasser oder eine wässrige Pufferlösung in den Reaktionsraum eingebracht. Insbesondere bei empfindlichen Templatpartikeln wie biologischen Aggregaten erfolgt die Zugabe des Waschmediums derart, daß das im Reaktionsraum befindliche Volumen des Mediums gemäß einem vorgegebenen Programm gesteuert wird, z.B. in Schritt (b) oder/und Schritt (d) im wesentlichen konstant bleibt.

Die Schritte (a) und (c) können jeweils im gleichen Reaktionsraum, aber auch in unterschiedlichen Reaktionsräumen durchgeführt werden. Die Filtrationsmembranen werden zweckmäßigerweise so gewählt, daß sie einerseits in der Lage sind, partikelförmige Templatmaterialien zurückzuhalten, aber andererseits eine rasche Entfernung des verbrauchten Reaktionsmediums ermöglichen. Beispiele für geeignete Filtermaterialien sind Polyamid, Cellulosenitrat und Celluloseacetat. Um bei empfindlichen Templatpartikeln eine Aggregatbildung oder/und ein Verstopfen des Filters zu vermeiden, wird das Verfahren unter Bedingungen durchgeführt, welche die Adhäsion von Templatpartikeln unterdrücken. So können gegebenenfalls für jeden Filtrationsschritt Membranen verwendet werden, die eine gleichsinnige Ladung wie die im jeweiligen Schritt verwendete Polyelektrolytspezies haben.

Die Filtration kann durch Anlegen eines Überdrucks im Reaktionsraum oder/und eines Vakuums im Filtratraum beschleunigt werden. Bei empfindlichen Templatpartikeln, insbesondere biologischen Aggregaten, wird die Filtration im wesentlichen ohne Druckdifferenz (Druckdifferenz ≤ ± 0,5 bar) zwischen Reaktionsraum und Filtratraum durchgeführt. Weiterhin ist ein Rühren des Reaktionsraumes zumindest während der Schritte (a) oder/und (c), insbesondere ein kontinuierliches Rühren während des gesamten Prozesses in vielen Fällen vorteilhaft.

Das erfindungsgemäße Membranfiltrationsverfahren ist kontinuierlich durchführbar, erlaubt die Produktion größerer Mengen an beschichteten Partikeln in kürzester Zeit, ist visuell kontrollierbar und verhindert weitestgehend die Aggregatbildung von Teilchen. Das Verfahren kann im großtechnischen Maßstab durchgeführt werden und kann aufgrund seiner Flexibilität an verschiedene Anforderungen der speziellen Partikel und Beschichtungssysteme adaptiert werden. Bei Verwendung löslicher Templatpartikel kann der Abbau der Kerne kontinuierlich im Anschluß an die Beschichtung erfolgen.

Anisotrope Hüllen, die unter Verwendung anisotroper Templatpartikel, z.B. biologischer Templatpartikel hergestellt wurden, erlauben in Verbindung mit z.B. Kristallisation oder/und Präzipitation die Herstellung von Kompositen mit anisotropen Eigenschaften. So können beispielsweise magnetische Ellipsoide hergestellt werden, z.B. durch Füllung mit magnetischen Partikeln oder/und durch Adsorption von magnetischen Nanopartikeln auf der Polyelektrolythülle. Im Magnetfeld zeigen diese anisotropen Partikel eine Orientierung, wodurch die optischen Eigenschaften einer Partikelsuspension rasant geändert werden können (magneto-optischer Schalter). Auf analoge Weise kann man mit ferroelektrischen Partikeln verfahren. Mit Hilfe dieser Teilchen kann man beispielsweise mit einem rotierenden Feld kleine Schaufelräder zum Pumpen erzeugen (Mikromechanik). Daneben können anisotrope Partikel durch Dissipation erwärmt werden. Dies kann zur Erzeugung extrem lokalisierter Wärmequellen genutzt werden, die man mit elektrischen bzw. mit magnetischen Feldern bewegen kann. Dies erlaubt die Erzeugung lokaler Hyperthermieffekte. Weiterhin können durch geordnete Ausrichtung anisotroper Partikel Kompositmaterialien mit hierarchischer Struktur und interessanten makroskopischen physikalischen anisotropen Eigenschaften erzeugt werden.

Wie bereits ausgeführt, kann die Permeabilität der Polyelektrolythülle durch Modifikationen, z.B. Aufbringen von Lipidschichten, gesteuert werden. Dies kann für pharmazeutischen Anwendungen genutzt werden, indem nach der Verkapselung polarer niedermolekularer Substanzen Lipide auf die Hülle aufgetragen werden, um auf diese Weise die Permeabilität der Hülle für die verkapselten Substanzen zu vermindern. Der verkapselte Stoff kann nur noch langsam durch die Lipidschicht mit einer über lange Zeit konstanten Rate austreten, was für pharmakologische Applikationen oftmals wünschenswert ist.

Durch die Verkapselung und anschließende Auflösung von Templaten können formgetreue dreidimensionale Abdrücke von Templatpartikeln hergestellt werden. Bei Blockvernetzung der Polyelektrolythüllen erhält man mesoporöse Materialien mit monodisperser formgetreuer Porenverteilung. Diese Materialien besitzen eine hohe innere Oberfläche verbunden mit hoher Festigkeit und machen sie zu hervorragenden Filtersubstanzen für industrielle Zwecke. Durch Auswahl der Template (Form und Größe) können mesoporöse Materialien mit vorgegebenen Poren hergestellt werden.

Natürlich kann durch Variation der für die Herstellung der Polyelektrolythülle verwendeten Materialien auch die Oberflächenchemie in weiten Bereichen variiert werden.

Schließlich können die Polyelektrolythüllen auch zur Erzeugung von pH-Gradienten zwischen dem inneren der Hülle und dem die Hülle umgebenden Volumen verwendet werden. Dieser pH-Gradient kann wiederum zu effizienten Beladungen der Hüllen mit Wirkstoffen ausgenutzt werden.

Die Erfindung wird durch die beigefügten Figuren und Beispiele weiter erläutert.
- Fig.1: stellt eine schematische Veranschaulichung eines Verfahrens zur Herstellung von Nano- und Mikrokapseln dar (nicht Gegenstand der Erfindung).
- Fig.2: zeigt die Schichtdicke als Funktion der Zahl der Schichten bei der Absorption von Poly(allylaminhydrochlorid) (PAH) und Poly(styrolsulfonat, Natriumsalz) (PSS) auf negativ geladene Polystyrol (PS)-Latexpartikel (nicht Gegenstand der Erfindung).
- Fig.3: zeigt ein SEM-Abbild (Rasterelektronenmikroskopie) einer Polyelektrolythülle mit neun Schichten (PSS/PAH)₄/PSS nach Auflösung des Kerns. Die äußere Schicht ist PSS (nicht Gegenstand der Erfindung).
- Fig.4: zeigt ein TEM-Abbild (Transmissionselektronenmikroskopie) einer Polyelektrolythülle mit neun Schichten (PSS/PAH)₄/PSS (nicht Gegenstand der Erfindung).
- Fig.5: zeigt atomkraftmikroskopische Abbildungen von PSS/PAH-Polyelektrolythüllen (nicht Gegenstand der Erfindung). Die Zahl der Polyelektrolythüllen beträgt in Fig.5(A) 3 PSS/PAH/PSS und in Fig. 5(B) 9 (PSS/PAH)₄/PSS].
- Fig.6: zeigt ein AFM-Abbild von PS-Latexteilchen mit 1,28 µm Durchmesser und einer Polyelektrolythülle mit sechs Schichten (PAH/PSS)₃. Die äußere Schicht ist PSS.
- Fig.7: zeigt normalisierte Lichtstreuungs-Intensitätsverteilungen von PAH/PSS-beschichteten PS-Latexpartikeln. Partikel mit 11 und 21 Schichten werden mit den unbeschichteten Partikeln verglichen.
- Fig.8: zeigt ein TEM-Abbild eines PS-Latexpartikels beschichtet mit vier Schichten PAH/PSS und anschließend drei Schichtpaaren bestehend jeweils aus einer Schicht Magnetit und einer Schicht PSS. Die Skala entspricht 200 nm.
- Fig. 9: zeigt ein AFM-Abbild einer Polyelektrolythülle erzeugt durch Beschichtung eines 3,7 µm MF-Latexpartikels mit 10 Schichten PSS/PAH und anschließende Auflösung des Templatpartikels.
- Fig.10: zeigt ein AFM-Abbild von Polyelektrolythüllen erhalten durch Beschichtung von Glutardialdehyd-fixierten humanen Erythrozyten mit zehn Schichten PSS/PAH und anschließende Auflösung des Templatpartikels. Die Skalenwerte der Achsen sind in µm und die Werte der Höhenskala in nm angegeben.
- Fig.11: zeigt ein AFM-Abbild von Polyelektrolythüllen erhalten durch Beschichtung von 3,7 µm MF-Latexpartikeln mit zehn Schichten Chitosan/Chitosansulfat und anschließende Auflösung des Templatpartikels.
- Fig.12: zeigt ein CLSM-Abbildung von Chitosan/Chitosansulfatmikrokapseln bestehend aus elf Schichten, wobei als äußerste Schicht FITC-markiertes PAH verwendet wurde.
- Fig.13: zeigt das Zetapotential von beschichteten und mit Chitosan/Chitosansulfat beschichteten 3,7 µm MF-Latexpartikeln.
- Fig.14: zeigt die Fluoreszenzintensität einer 6-CF als Fluoreszenzmarker enthaltenden Suspension von Polyelektrolythüllen gegenüber dem pH-Wert des umgebenden Mediums die durch HCl oder H-PSS mit dem jeweils angegebenen Molekulargewicht titriert wurde (nicht Gegenstand der Erfindung).
- Fig.15: zeigt die Beziehung des pH-Werts im Inneren der Kapseln zum pH-Wert des umgebenden Mediums, titriert mit H-PSS (MW 4200) in Abwesenheit von Salz (ausgefüllte Kreise) und in Gegenwart von 1 mM NaCl (leere Kreise). Die gestrichelte Linie zeigt die durch Titration der Kapseldispersion mit HCl erhaltene Kontrolle (nicht Gegenstand der Erfindung).

### Beispiele

### Beispiel 1 Herstellung von teilvernetzten Melaminformaldehyd-Templat-partikeln (nicht Gegenstand der Erfindung)

Monodisperse Melaminformaldehydpolymerpartikel können durch eine Polykondensationsreaktion aus Melaminformaldehydpräkondensaten im Größenbereich bis zu 15 µm hergestellt werden (vgl. DD 224 602). Die Größe der Partikel kann durch die Monomerkondensation, den pH-Wert, die Reaktionstemperatur und den Tensidzusatz beeinflußt werden. Bei den im Stand der Technik beschriebenen Verfahren werden hochvernetzte Partikel erhalten, die in den meisten organischen Lösungsmitteln, wie etwa Xylol, Toluol und Alkohol sowie in Säuren und Basen unlöslich sind. Zur Herstellung von auflösbaren, teilvernetzten Melaminformaldehyd-Templat-Partikeln wird das im Stand der Technik beschriebene Verfahren modifiziert, indem der Polykondensationsprozeß auf einer bestimmten Initialstufe der Reaktion unterbrochen wird. Dadurch werden in wäßrigen Medien lösliche Kerne erhalten. Der Abbruch der Reaktion kann durch rasche Temperaturabsenkung, durch Veränderung des pH-Werts in den alkalischen Bereich und durch Wahl geeigneter Präkondensate, insbesondere Tetramethylolmelamin, erreicht werden.

Die so erhaltenen Kerne können durch Säurezugabe und/oder durch bestimmte chemische Reaktionen, insbesondere Sulfonierung in wäßrigen Medien, aufgelöst werden. Als Sulfonierungsagentien können insbesondere Alkalisulfite, Alkalihydrogensulfite und andere wasserlösliche Salze der schwefligen Säure eingesetzt werden. Die Auflösbarkeit der Kerne kann durch den Zeitpunkt der Unterbrechung des Polykondensationsprozesses beeinflußt werden. Die Unterbrechung wird 1 min bis 3 h nach Start der Reaktion in Abhängigkeit der Reaktionsbedingungen und in Abhängigkeit der gewünschten Auflösbarkeit der Kerne durchgeführt. Die Auflösungsgeschwindigkeit kann weiterhin durch die Wahl von pH-Wert, Temperatur und Sulfonierungsreagens gesteuert werden. Somit ist es möglich, Kerne mit einer Auflösegeschwindigkeit von 0,1 s bis 10 h, wiederum in Abhängigkeit der Auflösungsbedingungen, zu erhalten. Diese auflösbaren Melaminformaldehydpartikel werden hierin als teilvernetzte Melaminformaldehydpartikel bezeichnet.

### Beispiel 2 Herstellung von leeren Polyelektrolythüllen unter Verwendung von Melaninformaldehydpartikeln als Templat (nicht Gegenstand der Erfindung)

### 2.1

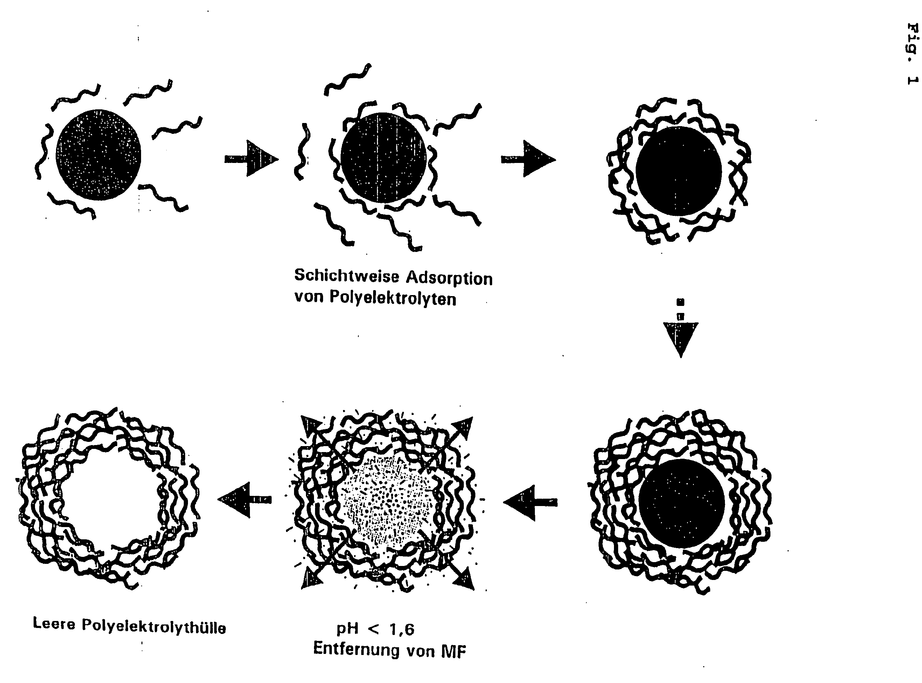

Auf wie in Beispiel 1 beschrieben hergestellte monodisperse, kolloidale, teilvernetzte Melaminformaldehydpartikel (MF) mit einem Durchmesser von 2,0 bzw. 3,3 µm werden schrittweise Polyelektrolyte aus verdünnten wäßrigen Lösungen aufgebracht (vgl.Fig.1). Die Polyelektrolytschichten werden durch alternierende Adsorption von entgegengesetzt geladenen Polyionen, beginnend mit der Adsorption eines negativ geladenen Polyanions (z.B. Polystyrolsulfonat, Natriumsalz; PSS) auf die positiv geladenen MF-Partikel aufgebracht. Typische Adsorptionsbedingungen waren 20 mM Polyelektrolyt (Konzentrationsangabe bezogen auf Monomer) 0,5 M NaCl bei Partikelkonzentrationen von 0,5 Gew.-%. Die Adsorptionsdauer betrug 20 min. Die MF-Partikel hatten eine Dichte von 1,5 g/cm³.

Nach Beendigung dieses Adsorptionszyklus wurde überschüssiger Elektrolyt durch wiederholte Zentrifugations/Waschzyklen entfernt. Dazu wurden die beschichteten Kerne bei einer Zentrifugationsgeschwindigkeit von 2000 U/min (unter Verwendung eines Eppendorf-Rotors) abgesetzt. Dann wurden drei Waschschritte mit entionisiertem Wasser vor der Zugabe des nächsten Polyelektrolyten durchgeführt, um die vollständige Entfernung von nichtadsorbiertem Polyelektrolyten sicherzustellen. Durch Wiederholung dieser Vorgehensweise kann die gewünschte Zahl von Polyelektrolytschichten aufgebracht werden.
Anschließend wurde der pH-Wert auf < 1,6 erniedrigt, wodurch die MF-Kerne innerhalb weniger Sekunden aufgelöst werden. Die Fragmente dringen durch die Poren der Hülle nach außen und können entfernt werden, so daß eine leere Polyelektrolythülle erhalten wird. Bei pH-Werten oberhalb 1,8 wird keine nachweisbare Auflösung der Kerne beobachtet.

### 2.2

100 µl einer 3%-igen Dispersion von teilvernetzten MelaminformaldehydPartikeln mit einer Partikelgröße von 3 µm werden mit 400 µl einer Lösung von 20 mono mM PSS in 0,5 M NaCl versetzt. Nach einer Einwirkzeit von 5 min unter leichtem Schütteln wird 1 ml reines Wasser zugesetzt. Nach Zentrifugation bei 2000 U/min wird der Überstand decantiert, das Sediment mit reinem Wasser aufgefüllt und die Zentrifugation wiederholt. Abermaliges Decantieren und ein weiterer Zentrifugationszyklus ergeben gereinigte, mit einer PSS-Schicht bedeckte MF-Partikel. In analoger Weise wird anschließend eine Poly(allylaminhydrochlorid)-Schicht (PAH) aufgebracht. Diese Zyklen werden abwechselnd in Abhängigkeit von der gewünschten Zahl der Schichten wiederholt. Nach dem Zentrifugationszyklus am Ende des Aufbaus der letzten Schicht werden 1 ml einer 0,1 N Salzsäure zugegeben. Nach etwa 5 min Schütteln erhält man eine klare Lösung, da die durch die Partikel verursachte Trübung der Lösung verschwunden ist. Anschließend zentrifugiert man für etwa 10 min bei 10 000 U/min. Bei dieser Zentrifugation scheidet sich ein feiner, leicht milchig wirkender Bodensatz ab, der die gebildeten Polyelektrolythüllen enthält. Bereits ein leichtes Schütteln nach Zusatz von Wasser ist ausreichend um die Hüllen zu resuspendieren. Nach zwei weiteren Zentrifugationsschritten erhält man eine gereinigte, 3%-ige Dispersion von sphärischen, monodispersen Polyelektrolythüllen in Wasser. Eine Probe dieser Hüllen kann durch Rasterelektronenmikroskopie, Transmissionselektronenmikroskopie und/oder Atomkraftmikroskopie untersucht werden.

### 2.3

1,59 mg Na-Polystyrolsulfonat (PSS) werden einer Dispersion von teilvernetzten Melaminformaldehydpartikeln in 0,5 M NaCl zugesetzt. Die MF-Dispersion enthält insgesamt 2,2 x 10⁸ Partikel. Nach zwanzigminütigem leichtem Schütteln werden 0,81 mg Polyallylaminhydrochlorid zugesetzt. Nach wiederum 20 min werden unter leichtem Schütteln 1,59 mg PSS zugegeben. Diese Vorgehensweise wird 5x mit jeweils einer PAH- und einer PSS-Zugabe wiederholt. Man erhält dadurch Melaminformaldehydpartikel, die mit 13 alternierenden Schichten bedeckt sind. Durch Zugabe von 10 ml 1 N-Salzsäure wird der pH-Wert erniedrigt, so daß sich die MF-Kerne auflösen. Durch die Zentrifugation bei 15 000 g für 15 min werden die Polyelektrolythüllen vom Überstand abgetrennt.

### Beispiel 3 Charakterisierung der Polyelektrolythüllen (nicht Gegenstand der Erfindung)

### 3.1 Abhängigkeit der Schichtdicke von der Anzahl der Schichten

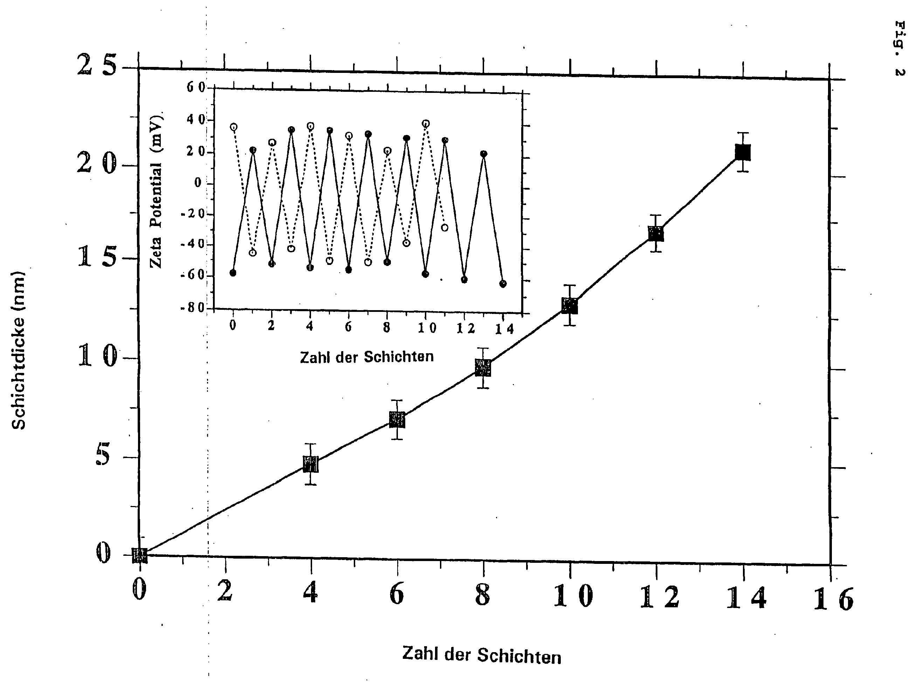

Auf negativ geladene Polystyrol (PS)-Latexpartikel wurden abwechselnd Schichten aus Poly(allylaminhydrochlorid) (PAH) und Poly(styrolsulfonat, Natriumsalz) (PSS) adorbiert. Die Schichtdicke wurde durch Einzelteilchen-Lichtstreuung gemessen. Der Intensitätsanstieg des gestreuten Lichts ist ein Maß für die adsorbierte Menge und wurde in die Schichtdicke unter Verwendung des Refraktionsindex der Polyeletrolytschichten konvertiert. Das Insert in Fig.2 gibt das aus elektrophoretischen Mobilitätsmessungen (Malvern Zetasizer 4) abgeleitete Zetapotential für die Adsorption von PAH und PSS auf Polystyrolpartikel (ausgefüllte Kreise) und für die Adsorption von PSS und PAH auf positiv geladene MF-Partikel (offene Kreise) an.

Das Zetapotential ist ein Maß für die effektive Ladungsdichte auf der Partikeloberfläche. Wie aus Fig.2 ersichtlich ist, tritt eine Umkehrung des Oberflächenpotentials mit der Adsorption jeder Polyelektrolytschicht auf die Polystyrol- bzw. MF-Partikel auf. Eine Umkehrung des Oberflächenpotentials begünstigt die anschließende Adsorption des entgegengesetzt geladenen Polyions.

Flugzeitmassenspektrometrische Untersuchungen haben gezeigt, daß bei der Auflösung der teilvernetzten MF-Templatpartikel bei einem pH-Wert < 1,6 MF-Oligomere gebildet werden, die hauptsächlich aus 5-10 Einheiten Tetramethylolmelamin bestehen. Diese MF-Oligomere haben eine charakteristische Querschnittsausdehnung von etwa 1 nm, wie durch molekulare Dynamiksimulationen bestimmt (unter Verwendung des Programms DISCOVERY). Diese Oligomere werden aus dem Kern ausgestoßen und permeiren durch Polyelektrolytschichten, die die Hülle bilden, und können schließlich von den leeren Schalen durch Zentrifugation abgetrennt werden. Dies bestätigt, daß die Hüllen für Moleküle mit einer Größe im Bereich von wenigen nm, insbesondere ≤ 10 nm, bevorzugt ≤ 5 nm, leicht permeabel sind.

### 3.2 Rasterelektronenmikroskopische Untersuchungen der Polyelektrolythüllen

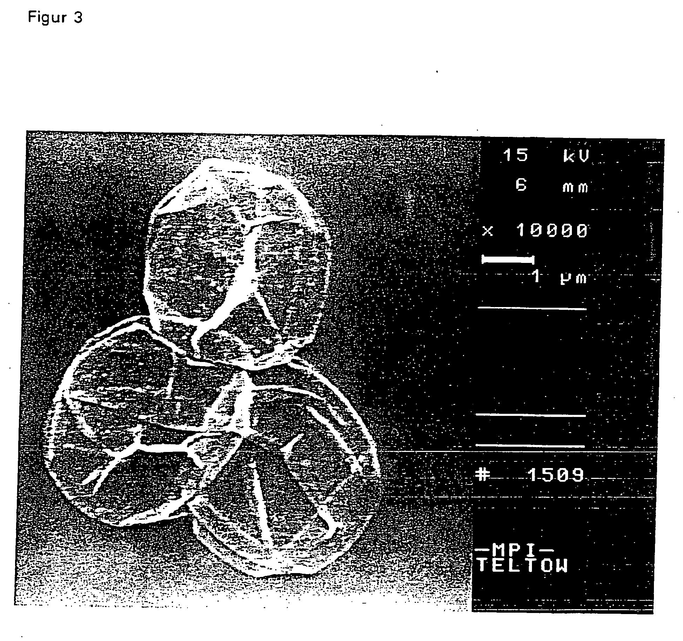

Die Polyelektrolythüllen wurden mittels Rasterelektronenmikroskopie (SEM) untersucht. Zunächst wurde ein MF-Kern mit einem Durchmesser von 3,3 µm mit 9 Polyelektrolytschalen (PSS/PAH)₄/PSS beschichtet. Die äußerste Schicht ist PSS. Nach Auflösung des MF-Kerns wurden die erhaltenen Kapseln mit SEM untersucht. Wie aus Fig.3 ersichtlich, liegen die Durchmesser im Bereich von 4,0 ± 0,5 µm. Die Schalen werden durch eine starke elektrostatische Anziehung an die positiv geladene, Poly(ethylenimin)-beschichtete Glasoberfläche immobilisiert. Weiterhin tritt während der Untersuchung ein gewisses Ausmaß an Austrocknen der Kapseln ein. Dies führt dazu, daß die Hülle faltig wird. Wie jedoch aus Fig.3 ersichtlich ist, sind keine Löcher oder Rißspuren in den Hüllen zu finden.
Die SEM-Messungen wurden unter Verwendung eines Zeiß-DSM40-Instruments durchgeführt, welches bei einer Beschleunigungsspannung von 15 KeV betrieben wurde. Die Proben wurden durch Aufbringen eines Tropfens einer die Hüllen enthaltenden Lösung auf Poly(ethylenimin)-beschichtetes Glas hergestellt. Nachdem sich die Hüllen auf den Glasträgern abgesetzt hatten, wurden sie gründlich mit Wasser gespült und vorsichtig unter einem Stickstoffstrom getrocknet.

### 3.3 Transmissionselektronenmikroskopie (TEM)

Auf MF-Templatpartikel mit einem Durchmesser von 2 µm wurden neun Schichten Polyelektrolyt (PSS/PAH)₄/PSS aufgebracht. Anschließend wurden die Templatpartikel aufgelöst. Die Proben wurden mit Glutardialdehyd, OsO₄ und K₂Cr₂O₇ fixiert, in Ethanol/Aceton dehydriert, in ein Epon 812/Araldit M-Harz eingebettet und in einem Ofen für zwei Tage polymerisiert. Dünnschnitte (80 bis 100 nm) wurden unter Verwendung eines Reichert-Ultratom angefertigt und mit Uranylacetat und Bleicitrat eingefärbt. Die Messungen wurden auf einem JEOL 100 B Elektronenmikroskop durchgeführt.

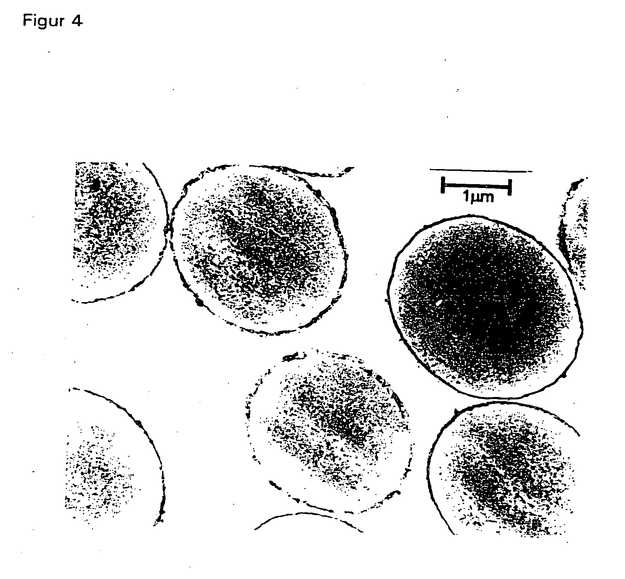

Wie aus Fig.4 ersichtlich, kann die angefärbte Polyelektrolytschicht, die das weniger angefärbte Zellinnnere umgibt, deutlich identifiziert werden. Die homogene Form der Hüllen zeigt, daß die hergestellten Kapseln sowohl den Durchmesser als auch die sphärische Form der Templatpartikel beibehalten, unter der Voraussetzung, daß die innere wäßrige Lösung nicht entfernt wird. Weiterhin ist zu erkennen, daß die Dicke der aus neun Schichten bestehenden Polyelektrolythülle in der Größenordnung von 20 nm ist. Dieser Wert stimmt mit den in Fig.2 gezeigten Daten für Polyelektrolyt-beschichtete Polystyrolpartikel überein. Daraus kann man schließen, daß die Art der Templatpartikel die Dicke der Polyelektrolytschichten nicht wesentlich beeinflußt. Aus dem TEM-Abbild ist auch ersichtlich, daß die Polyelektrolythüllen weder Risse noch Löcher aufweisen.

### 3.4 Atomkraftmikroskopische (AFM) Untersuchungen

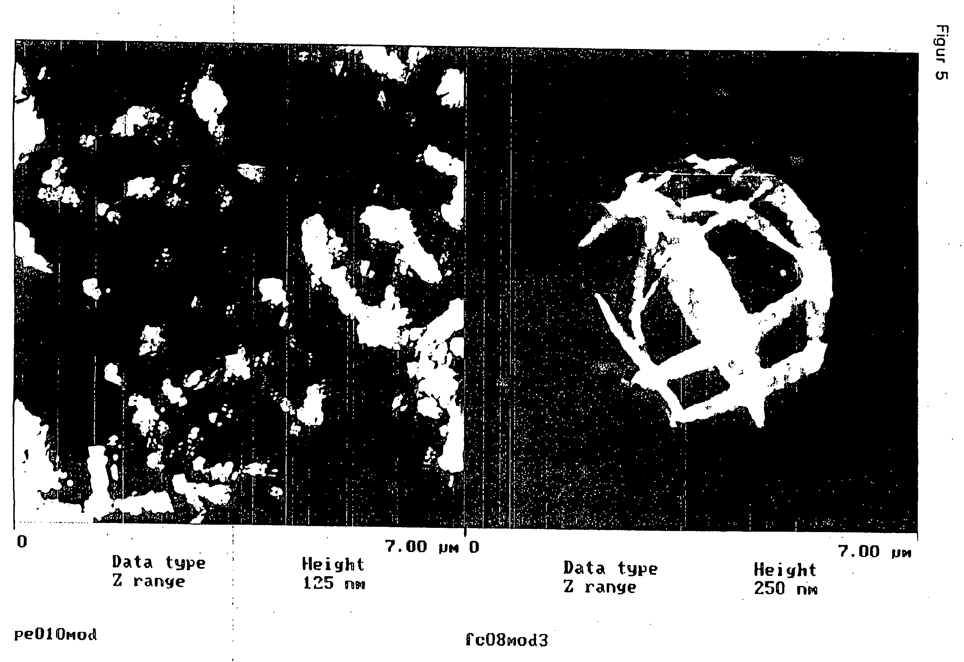

PSS/PAH-Polyelektrolythüllen wurden unter Verwendung von MF-Templatpartikeln mit einem Durchmesser von 3,3 µm wie oben beschrieben hergestellt. Die Zahl der Polyelektrolytschichten betrug 3 PSS/PAH/PSS (Fig.5(A)) bzw. 9 (PSS/PAH)₄/PSS (Fig.5(B)). Diese Kapseln wurden mit AFM im Tapping Mode (TM) untersucht. Fig.5 zeigt, daß die dreidimensionalen Polyelektrolythüllen durchgehende Folien sind, die Falten aufweisen, die von der Verdampfung des wäßrigen Inneren resultieren. Wie zu sehen ist, steigt die Höhe der Kapseln mit ansteigender Schichtzahl an. Die maximale Höhe der getrockneten Hüllen in Abbildung A liegt in der Größenordnung von 50 nm und in Fig.5(B) in der Größenordnung von 100 nm.

### Beispiel 4 Herstellung von trägerfixierten Polyelektrolythüllen (nicht Gegenstand der Erfindung)

Ein sorgfältig gereinigter Glasträger wird 5 min in eine wäßrige Lösung von 0,5 mg/ml Polyethylenimin eingetaucht. Danach bläst man den Glasträger in einem Stickstoffstrom trocken. 100 µl einer 3%-igen Dispersion aus teilvernetzten Melaminformaldehydpartikeln mit einer Partikelgröße von 1 µm Durchmesser werden mit 400 µl einer 20 mono-mM Na-Poly(styrolsulfonat)-lösung NaCl versetzt. Nach 5 min unter leichtem Schütteln wird 1 ml reines Wasser zugesetzt. Nach Zentrifugation bei 2000 U/min wird der Überstand decantiert, das Sediment mit reinem Wasser aufgefüllt und die Zentrifugation wiederholt. Nach abermaligem Decantieren und einem weiteren Zentrifugationszyklus erhält man mit einer PSS-Schicht bedeckte MF-Partikel. Anschließend gibt man 400 µl einer 20 mono-mM-Polydiallyldimethylammoniumchloridlösung in 0,5 M NaCl zu den Partikeln und inkubiert für 20 min Diesen Vorgang wiederholt man ein zweites Mal. Anschließen werden die Partikel wiederum wie oben beschrieben mit PSS beschichtet und dreimal zentrifugiert. Das Sediment wird in 0,5 ml reinem Wasser redispergiert und auf den Glasträger aufgebracht. Nach 5 min taucht man den Glasträger für 5 min in eine 0,1 N Salzsäurelösung. Danach taucht man das Glasplättchen ohne Zwischentrocknung für jeweils 5 min dreimal in reines Wasser. Danach wird das Glasplättchen in einem leichten Stickstoffstrom getrocknet. Als Resultat erhält man dichtgepackte, auf einem Polyethylenimin-beschichteten Glasträger fixierte Polyelektrolythüllen, die aus 5 Schichten bestehen.

### Beispiel 5 Einschluß von Wirkstoffen in Polyelektrolythüllen (nicht Gegenstand der Erfindung)

100 µl einer 2%-igen Dispersion aus teilvernetzten Melaminformaldehydpartikeln mit einer Partikelgröße von 0,9 µm Durchmesser werder mit 400 µl einer 0,5 M NaCl-Lösung, pH 6, versetzt, die 0,5 mg/ml Polymilchsäure enthält. Nach 5 min unter leichtem Schütteln wird 1 ml Wasser zugesetzt. Nach Zentrifugation bei 2000 U/min (5 cm Rotorradius) wird der Überstand decantiert, Wasser nachgefüllt und die Zentrifugation wiederholt. Nach abermaligem Decantieren und einem weiteren Zentrifugationszyklus werden mit einer Polymilchsäureschicht bedeckte Melaminpartikel erhalten. Diese werden mit 0,4 ml einer 1 mg/ml Lysozymlösung bei pH 6,0 versetzt und für 20 min bei leichtem Schütteln inkubiert. Anschließend wird dreimal in Wasser gewaschen. Bei pH 6 wird eine weitere Polymilchsäureschicht wie oben beschrieben aufgebracht. Anschließend wird eine Poly(allylaminhydrochlorid)schicht (PAH) aufgebracht, danach weitere Schichten in der Folge PSS/PAH/PSS.

Anschließend überführt man die Partikel in eine 0,1 N Salzsäurelösung. Nach wenigen Sekunden bilden sich unter Auflösung der Kerne und der zwei Polymilchsäureschichten mit Lysozym gefüllte Polyelektrolythüllen aus. Diese werden zweimal bei 15 000 g in reinem Wasser zentrifugiert. Der Überstand wird jeweils verworfen. Man erhält als Sediment konzentrierte, mit Lysozym, einem Protein, gefüllte Kapseln, die eine Polyelektrolythülle aus 4 Schichten aufweisen. Andere biologische Makromoleküle können auf ähnliche Weise verkapselt werden.

### Beispiel 6 Herstellung von leeren Polyelektrolythüllen unter Verwendung biologischer Partikel als Templat (nicht Gegenstand der Erfindung)

Rinder- oder Humanerythrozyten werden mit Glutadialdehyd in einer Konzentration von 2% fixiert. Nach 60 min Einwirkzeit bei 20°C wird die Lösung abzentrifugiert und die Erythrozyten werden viermal in bidestilliertem Wasser gewaschen. Anschließend werden die fixierten Erythrozyten mit ungepufferter 154 mM NaCl-Lösung aufgefüllt.

Zur Beschichtung werden 4 ml Lösung mit einer Konzentration von 0,5 g/dl PAH und 0,5 M NaCl bei einer Erythrozytenkonzentration von ca. 2,5% (v/v) angesetzt. Nach 10 min Einwirkzeit bei 20°C werden die Erythrozyten abzentrifugiert und zweimal in einer 154 mM NaCl Lösung gewaschen. Anschließend werden 4 ml Lösung mit einer Konzentration von 0,5 g/dl PSS und 0,5 m NaCl und einer Erythrozytenkonzentration von ca. 2,5% (v/v) angesetzt. Nach 10 min Einwirkzeit bei 20°C werden die Erythrozyten abzentrifugiert und zweimal in einer 154 mM NaCl-Lösung gewaschen. Das Aufbringen von PAH und PSS Schichten kann beliebig oft wiederholt werden.

Die Auflösung des Templats kann in einer 1,2%-igen NaOCl Lösung erfolgen. Ebenso geeignet sind handelsübliche Deproteinisierungsmittel (Medical Instruments) oder Abflußreiniger (z.B. Chlorix). Die Einwirkzeit beträgt ca. 20 min bei 20°C und läßt sich optisch über die verschwindende Trübung der Lösung kontrollieren. Die verbleibenden Polymerhüllen werden anschließend in NaCl-Lösung gewaschen.

Auf analoge Weise können auch E.coli oder Hefezellen beschichtet werden. Auch nichtfixierte Zellen können beschichtet werden.

### Beispiel 7 Abscheidung von Lipidschichten auf Polyelektrolythüllen (nicht Gegenstand der Erfindung)

Zur Abscheidung von Lipidschichten auf Polyelektrolythüllen wurden zwei unterschiedliche Verfahren verwendet.

### 7.1

200 µl einer Suspension von Polyelektrolythüllen werden durch wiederholtes Waschen in Methanol resuspendiert. Nach dem dritten Waschen werden anstelle von reinem Methanol 500 µl einer Lipidlösung von z.B. 1 mg/ml Dipalmitoylphosphatidinsäure (DPPA) oder Dipalmitoylphosphatidylcholin (DPPC) in Methanol dem Sediment zugesetzt. Die Hüllen werden in dieser Methanol-Lipidlösung resuspendiert und die Suspension bei einer Temperatur von 90°C in einem Wasserbad gehalten. Der verdampfende Methanol wird durch tropfenweise Zugabe von Wasser in Portionen von jeweils 20 µl ersetzt. Der Austausch von 700 µl Methanol gegen Wasser dauert etwa 30 min.

Nach Beendigung der Verdampfung wird die Hüllensuspension dreimal mit Wasser gewaschen und wiederholt zentrifugiert. Durch 20 min Zentrifugation bei 25 000 Upm können die Lipid-beschichteten Hüllen sedimentiert werden.

### 7.2

Dispersionen von DPPA oder 90% DPPC und 10% DPPA mit einer Konzentration von 1 mg Lipid/ml in Wasser werden durch Ultraschallbehandlung hergestellt. 500 µl der resultierenden Dispersion von Lipidvesikeln werden auf 200 µl einer konzentrierten Hüllensuspension gegeben. Nach 30 min werden die Proben 20 min bei 25 000 Upm zentrifugiert. Der Überstand wird entfernt und durch Wasser ersetzt. Diese Prozedur wird dreimal wiederholt. Dabei wird eine konzentrierte Suspension von Lipid-beschichteten Hüllen erhalten.

### Beispiel 8 Einschluß von organischen Lösungsmitteln in Polyelektrolythüllen (nicht Gegenstand der Erfindung)

Eine wässrige Suspension von Polyelektrolythüllen wird 5 min bei 3000 Upm zentrifugiert. Nach Entfernen des Überstands wird Methanol zugegeben. Die Hüllen werden resuspendiert und 10 min lang bei 4000 Upm zentrifugiert. Erneut wird der Überstand entfernt, Methanol zugegeben und die Probe unter denselben Bedingungen wie zuvor zentrifugiert. Diese Prozedur wird dreimal wiederholt. Nach der letzten Zentrifugation mit Methanol wird der Überstand durch Hexanol ersetzt. Die Hüllen werden resuspendiert und 10 min bei 5000 Upm zentrifugiert. Diese Prozedur wird wiederum dreimal wiederholt.

Zum Einschluß von Octanol, Octan oder Decan in die Hüllen wird eine ähnliche Prozedur verwendet, wobei als Ausgangsmaterial die in einer Hexanollösung vorliegenden Hüllen verwendet werden. Die Zentrifugationsgeschwindigkeit wird für Octanol und Octan auf 7000 Upm (10 min) und für Decan auf 7500 Upm (10 min) erhöht.

Schließlich wird das resultierende Sediment in Wasser resuspendiert. Die Hüllen verbleiben in der wässrigen Phase, während die noch vorhandenen Spuren des Lösungsmittels im Sediment zwischen den Hüllen eine zweite organische Phase bilden. Durch Verwendung von Fluoreszenzmarkern für die organische und die wässrige Phase kann mittels konfokaler Mikroskopie gezeigt werden, daß die Hüllen mit organischem Lösungsmittel gefüllt sind.

Die beschriebene Prozedur ermöglicht die Herstellung einer hochstabilen Emulsion von nichtpolaren Flüssigkeiten in Wasser. Als Folge der Monodispersität der ursprünglichen Hüllen ist die erzeugte Emulsion ebenso monodispers. Ein weiterer Vorteil ist, daß selbst die Form der einzelnen Tröpfchen - abhängig vom verwendeten Templat - reguliert werden kann. Dies ermöglicht die Herstellung von Emulsionen mit Oberfläche:Volumen-Verhältnissen, die von denjenigen einer Kugel verschieden sind.

### Beispiel 9 Präzipitation und Kristallisation in Polyelektrolythüllen (nicht Gegenstand der Erfindung)

Die leeren Polyelektrolythüllen können auch zur kontrollierten Präzipitation oder Kristallisation organischer oder anorganischer Materialien verwendet werden. Hierzu werden Polyelektrolythüllen in einer 30 mM 6-Carbofluorescein (6-CF) Lösung bei pH 7 inkubiert. Anschließend wird der pH-Wert der Lösung rasch auf einen Wert von 3,5 verändert, bei dem 6-CF weitgehend unlöslich wird. Nach Inkubation über 1 bis 12 h wird eine Mischung von vollständig mit 6-CF gefüllten und leeren Hüllen erhalten. In weiteren Experimenten konnte Rhodamin B in Polyelektrolythüllen durch Erhöhung des pH-Werts präzipitiert werden.

Die Präzipitation von Wirkstoffen kann auch durch andere Maßnahmen, z.B. Lösungsmittelaustausch, Salzfällung, etc. ausgelöst werden. Diese Ergebnisse zeigen, daß die Polyelektrolythüllen als Template für Kristallisations- oder Präzipitationsprozesse dienen können, wodurch eine Kontrolle der Größe und Form von durch die Reaktion entstandenen kolloidalen Teilchen ermöglicht wird.

### Beispiel 10 Polymerisation in Polyelektrolythüllen (nicht Gegenstand der Erfindung)

Eine 3% Lösung von Diallyldimethyldiammoniumchlorid (DADMAC) wird in einer 2%-igen Suspension von Polyelektrolythüllen mit dem Polymerisationsinitiator Natriumperoxodisulfat (30 mg/100ml) versetzt und 9,5 h bei 70°C polymerisiert. Durch Zentrifugation wird das in der Volumenphase synthetisierte Polymer PDADMAC abgetrennt. Durch Behandlung mit 100 mM 6-CF, das an die Aminogruppen von PDADMAC bindet, kann das Vorhandensein von an die negativ geladenen Kapselwände adsorbiertem Polymer und im Inneren der Kapsel befindlichem Polymer nachgewiesen werden.

Aus 9 Schichten bestehende Polyelektrolythüllen ([PSS/PAH]₄PSS) werden auf Humanerythrozyten abgeschieden und die Templatpartikel entfernt. Anschließend wird eine weitere Schicht PAH aufgetragen. Die Kapseln werden zur radikalischen Polymerisation von Acrylsäure zu Polyacrylsäure verwendet. Hierzu wird eine 3%ige Monomerlösung in einer 2%igen Kapselsuspension mit dem Initiator Natriumperoxodisulfat (30 mg/100 ml) versetzt und 9,5 h bei 70°C polymerisiert. Durch Zentrifugation wird die in der Volumenphase synthetisierte Polyacrylsäure entfernt. Nach Zugabe von 100 mM Rhodamin B, das selektiv an anionische Gruppen bindet, kann das Vorhandensein von Polyacrylsäure adsorbiert an die negativ geladenen Kapselwände, aber auch im Inneren der Kapseln nachgewiesen werden. Die Adsorption von Acrylsäure an Kapselwände kann durch Verwendung von Kapseln mit einer äußeren negativen Ladung verhindert werden.

### Beispiel 11 Kolloidale Stabilisierung von mit organischen Lösungsmitteln gefüllten Polyelektrolythüllen (nicht Gegenstand der Erfindung)

Polyelektrolythüllen in wässriger Lösung werden mit DPPA (unter Zusatz von 5% markiertem DPPC) beschickt. Die wässrige Dispersion wird mit Pentanol, Octanol oder Decan gemischt. Die Proben werden für 2 min bei 17.000 Upm zentrifugiert. Die Mischung trennt sich in zwei Phasen auf, wobei die Hüllen an der Zwischenschicht zwischen der wässrigen und der organischen Phase lokalisiert sind. Die wässrigen Phase wird extrahiert und die Probe dreimal mit dem organischen Lösungsmittel gewaschen.

Durch konfokale Mikroskopie kann nachgewiesen werden, daß die Lipide selbst nach Kontakt mit dem organischen Lösungsmittel an den Hüllen verbleiben und daß im Inneren der Hülle eine wäßrige Phase verkapselt ist. Hierzu werden die Kapseln vor dem Aufbringen der Lipidschicht 1 h in einer 0,1 mM 6-CF-Lösung inkubiert und die Probe nach Aufbringen der Lipide viermal mit Wasser zur Entfernung von überschüssigen Lipiden und 6-CF gewaschen. Ein 12 h nach der Präparation aufgenommenes konfokales Bild zeigt, daß die wässrige 6-CF-Lösung immer noch innerhalb der Hüllen verkapselt ist. Das organische Lösungsmittel zeigt keine Fluoreszenz.

Diese Ergebnisse zeigen, daß die Polyelektrolythüllen zur Verkapselung organischer Lösungsmittel in Wasser und auch umgekehrt zur Verkapselung einer wässrigen Phase im organischen Medium verwendet werden können. Somit können stabile Öl-in-Wasser- und Wasser-in-Öl-Emulsionen ohne Verwendung oberflächenaktiver Mittel erzeugt werden.

### Beispiel 12 Herstellung von Polyelektrolythüllen durch Membranfiltration

### 12.1 Materialien

Als Templatpartikel wurden geladene Polystyrol-Latexteilchen mit einem Durchmesser von 640 nm, die nach der Methode von Furosawa et al. (Colloid-Z. Z. Polym. 250 (1972), 908) hergestellt wurden, teilvernetzte Melaminformaldehydpartikel mit Durchmessern von 3,7 µm und 5 µm sowie mit Glutardialdehyd fixierte humane Erythrozyten verwendet. Als Beschichtungssubstanzen werden Natriumpoly(stryrolsulfat) PSS (MW 70.000), Poly(allylaminhydrochlorid) PAH (MW 8.000 bis 11.000), Poly(diallyldimethylammoniumchlorid) PADMAC (MW 100.000), Chitosan (MW 200.000 bis 300.000), Chitosansulfat (MW 200.000 bis 300.000) und Magnetit-partikel verwendet. Hierzu werden wässrige Lösungen von 1 oder 2 mg/ml PSS, PAH oder PDADMAC in 0,5 M NaCl hergestellt. Chitosansulfat wird als Lösung mit einer Konzentration von 1 mg/ml in 0,5 M NaCl hergestellt. Chitosan wird in einer Konzentration von 1 mg/ml in 0,5 M NaCl unter Zusatz von 0,3% (v/v) Essigsäure aufgelöst.
Es wird die Vakuumpumpe SM 16692 (Sartorius AG, Göttingen, Deutschland) zur Membranfiltration verwendet, mit der ein Vakuum von etwa 100 mbar und ein Überdruck bis zu 3 bar erzeugt werden kann. Zur Vakuumfiltration wird die Polycarbonatfiltrationseinheit SM 16510 (Sartorius) und zur Druckfiltration die Einheit SM 16526 (Sartorius) verwendet.

Es werden Membranfilter mit einem Durchmesser von 47 mm der folgenden Typen verwendet: Sartolon Polyamid SM 25007-047 N (0,2 µm), Sartolon Polyamid SM 25006-047 N (0,45 µm), Celluloseacetat SM 11104-047 N (0,8 µm) und Cellulosenitrat SM 11306-100 N (0,45 µm).

### 12.2 Methoden

Polystyrol- und Melaminformaldehyd-Latexsuspensionen werden in einer Konzentration von 1 bis 30 % (v/v) eingesetzt. Die Konzentration der Erythrozytensuspension sollte etwa 10% (v/v) nicht übersteigen. Das Volumen der im ersten Adsorptionsschritt verwendeten Partikelsuspensionen ist zwischen 10 ml und 50 ml. Die Adsorptionsdauer ist immer 5 min. Danach werden die Partikel mit Wasser gewaschen.

Die Membranfiltration kann als Vakuumfiltration, Druckfiltration und Filtration ohne Druckveränderung durchgeführt werden. Während der Polyelektrolytadsorption wird vorzugsweise ein schwacher Unterdruck in der Inkubationskammer bezüglich der unteren Filtratkammer angelegt, um einen Verlust des Inkubationsmediums und des Polyelektrolyten während der Adsorption zu vermeiden.

Ein Gesichtspunkt bei der Auswahl des Membranfilters ist das Ladungsvorzeichen des zu adsorbierenden Polyelektrolyten. Im Falle von Polykationen (PAH, Chitosan) sind beispielsweise Polyamidfilter geeignet. Im Falle von Polyanionen können Celluloseacetat oder Cellulosenitratfilter verwendet werden. Auf diese Weise kann Verstopfen des Filters durch Polyelektrolytadsorption gering gehalten werden.

Zweckmäßigerweise wird die Filtration unter Bedingungen durchgeführt, bei denen ein Entstehen oder Zusammenbacken des Filterkuchens vermieden bzw. begrenzt wird. Die Adhäsion von PS und MF-Latexpartikel mit einer äußeren Schicht von PSS oder PAH an die Filteroberflächen ebenso wie deren Aggregations- oder/und Flokkulationsneigung ist gering. Die Filtration kann daher bis zu hohen Teilchenkonzentrationen (20%) ohne Unterbrechung oder Ersetzen des abfiltrierten Suspensionsmediums durch das Waschmedium durchgeführt werden. Im Falle von Erythrozyten muß während der Abscheidung der ersten vier oder fünf Schichten vorsichtig gearbeitet werden, um eine Aggregation zu verhindern. Die Suspension sollte daher nicht auf Konzentrationen von mehr als etwa 5 bis 10% (v/v) eingeengt und die Bildung eines Filterkuchens soweit wie möglich vermieden werden. Nach Abschluß der ersten Adsorptionszyklen nimmt die Aggregationsneigung sowie die Neigung zur Adhäsion an den Filter ab. Eine gegebenenfalls zwischenzeitlich auftretende Flockung während der Zugabe eines Polyelektrolyten kann bei laufendem Prozeß durch Zugabe eines entgegengesetzt geladenen Polyelektrolyten, z.B. des nachfolgenden Polyelektrolyten ohne Schädigung des Produkts wieder aufgelöst bzw. gebrochen werden.

Ähnliche Ergebnisse werden mit dem Chitosan/Chitosansulfat-System erhalten. Eine weitgehend vollständige Unterdrückung der Aggregatbildung kann durch Rühren erreicht werden.

### 12.3 Ergebnisse

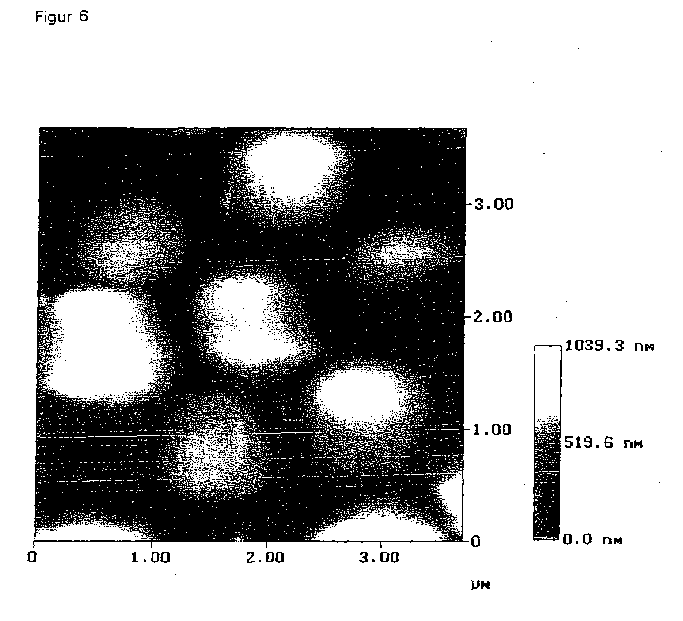

In Figur 6 ist eine AFM-Abbildung von PS-Latexoberflächen nach Aufbringen von 3 PAH/PSS-Schichtpaaren gezeigt. Die Oberflächen sind glatt und es sind keine Polyelektrolytaggregate zu erkennen. Diese Partikel wurden durch Vakuummembranfiltration mit einem Saugdruck von etwa 100 mbar und unter Verwendung von 450 nm Cellulosenitrat-Membranfiltern hergestellt. Ebenso können Polyamidfilter oder abwechselnd negativ geladene Celluloseacetat- und positiv geladene Polyamidfilter verwendet werden.

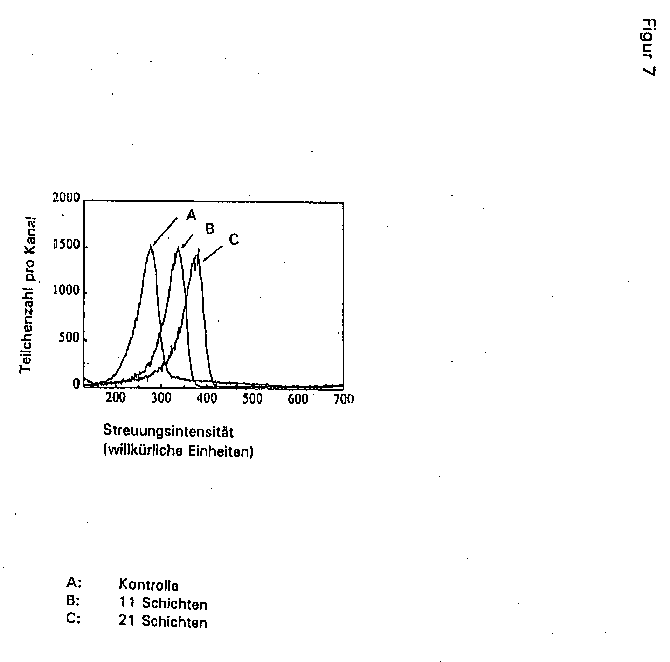

In Figur 7 wird das Wachstum der Hüllendicke an der Oberfläche von 640 nm PS-Latexpartikeln durch Einzelpartikel-Lichtstreuung nach der bei Lichtenfeld et al. (Colloid Surfaces A 104 (1995), 313) beschriebenen Methode bestimmt. Die Streulichtintensität nimmt mit der Teilchengröße zu. Es sind die Ergebnisse von Teilchen mit 11 bzw. 21 PAH/PSS Schichten im Vergleich zu unbeschichteten Teilchen gezeigt.

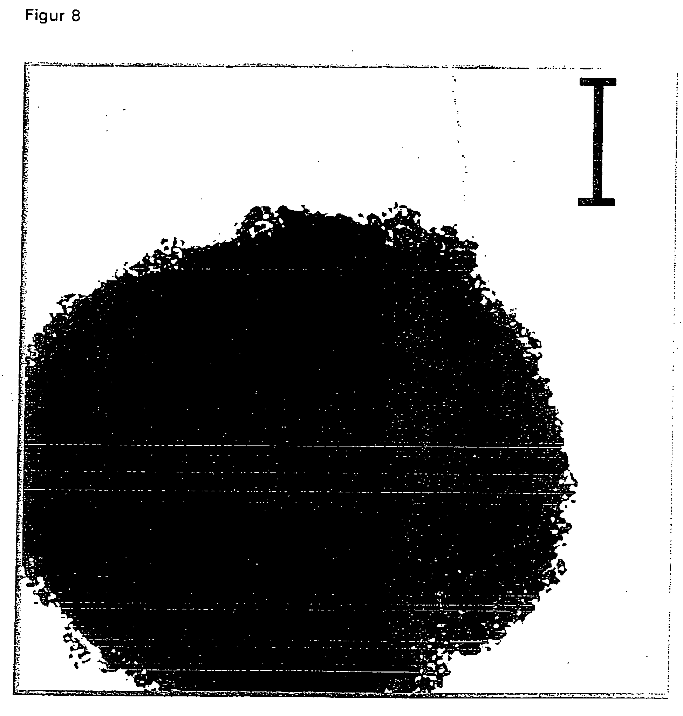

Das TEM-Bild eines mit einer gemischten Schichten aus Polyelektrolyt- und Magnetitteilchen, die durch Präzipitation von Eisen (II)- und Eisen (III)-Salzen in Ammoniumhydroxydlösung und Stabilisierung durch HCl erzeugt wurden (Massart und Cabuil, J. D. Chemie Physique 84 (1987), 967), ist in Figur 8 gezeigt. Zunächst werden zwei PAH/PSS Doppelschichten adsorbiert. Danach werden drei Magnetit/PSS Doppelschichten adsorbiert. Die Filtratlösung war jeweils ungefärbt und klar. Dies bedeutet eine vollständige Adsorption des Magnetits. An der Oberfläche gebildete Magnetitaggregate sind deutlich erkennbar.

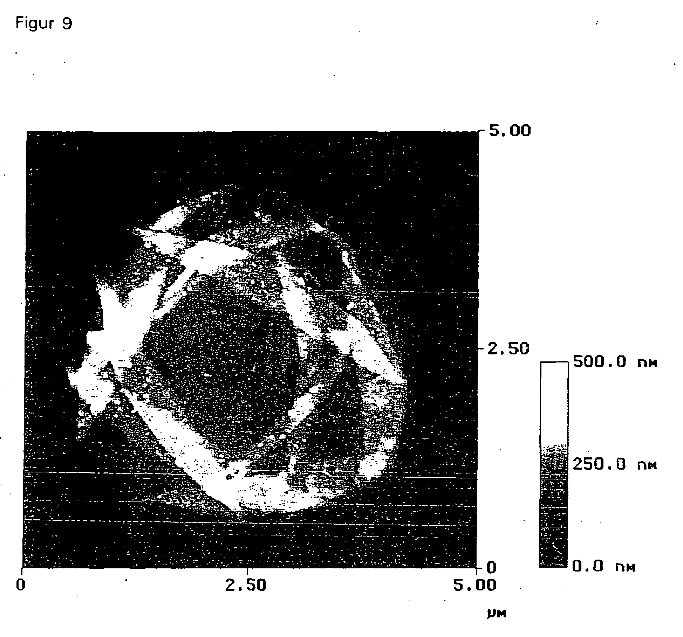

Bei Verwendung von auflösbaren MF-Partikeln als Templat werden ebenfalls gute Ergebnisse bei der Membranfiltration erzielt. Mikrokapseln mit 10 PAH/PSS Schichten auf 3,7 µm MF-Partikeln sind im AFM-Bild von Figur 9 gezeigt. Die Templatpartikel wurden im Citratpuffer pH 1,4 aufgelöst. Die Morphologie der flachen Hülle ist deutlich zu erkennen.

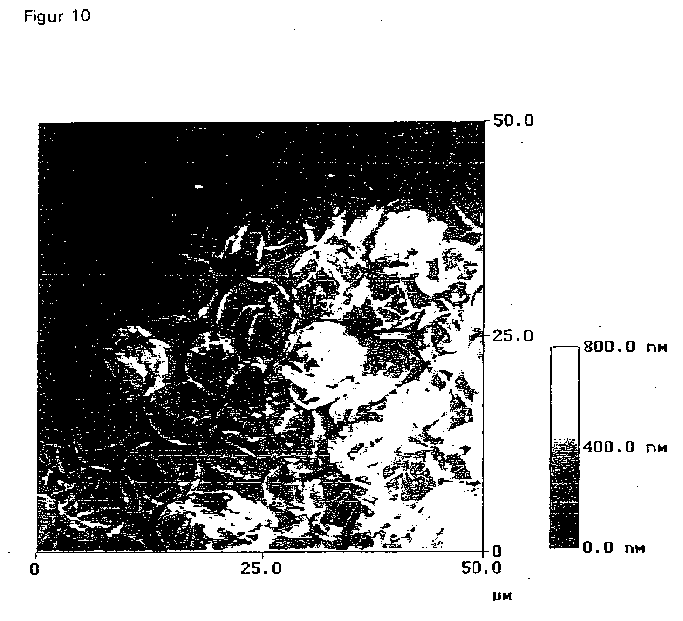

In Figur 10 ist ein AFM-Bild von PAH/PSS Mikrokapseln mit 10 Schichten gezeigt, die an der Oberfläche von Glutaraldehyd fixierten humanen Erythrozyten abgeschieden wurden. Trotz der negativen Ladung der Zellen ist es günstig mit einer PSS-Adsorptionsschicht zu beginnen. Auf diese Weise kann während der ersten Adsorptionsschritte das Ausmaß der Aggregation vermindert werden. Die Filtration sollte unter milden Druckbedingungen (kein oder nur geringer Über- oder Unterdruck) erfolgen, um die Bildung von Filterkuchen zu vermeiden. Ähnliche Vorsichtsmaßnahmen sind bei Verwendung von PDADMAC als Polykation zu beachten. Unter druckfreien Bedingungen werden auch in solchen empfindlichen Systemen hervorragende Ergebnisse erzielt.

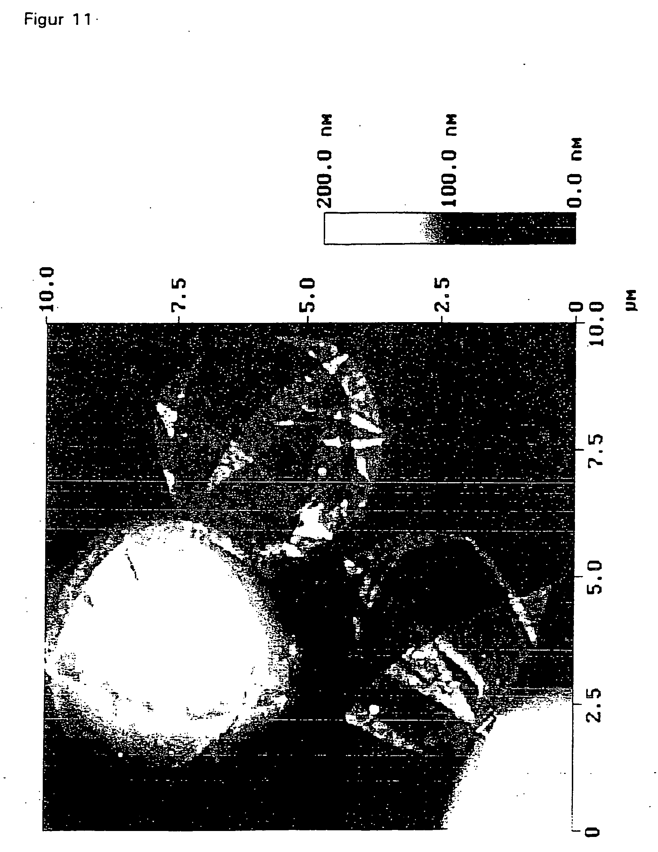

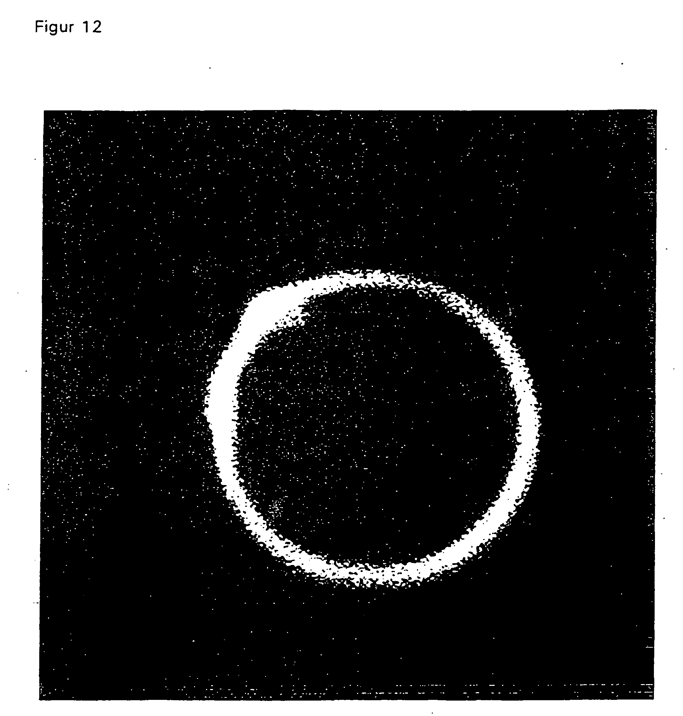

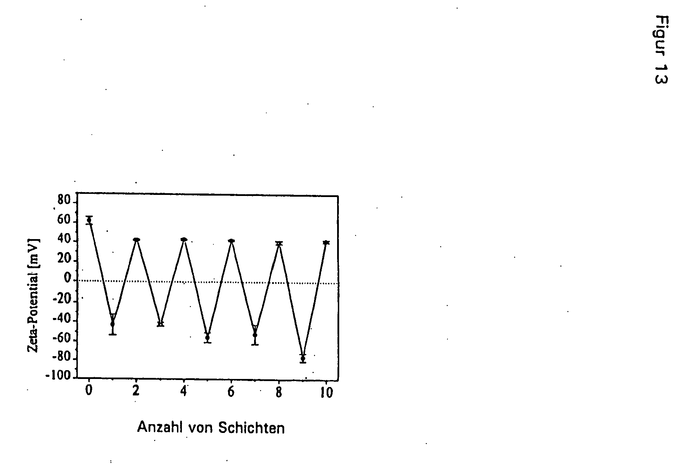

In den Figuren 11 bis 13 sind Ergebnisse dargestellt, die unter Verwendung von Chitosan/Chitosansulfat als Polyelektrolytpaar erhalten wurden. Figur 11 ist ein AFM-Bild von mit 10 Schichten belegten MF-Partikeln nach Auflösung des Kerns. Um die Untersuchung der Hüllmorphologie zu erleichtern wurde mit FITC markiertes PAH als elfte Schicht aufgebracht und die Hülle durch konfokale Laserrastermikroskopie untersucht. Das Ergebnis ist in Figur 12 gezeigt. In Figur 13 sind die Zetapotentiale von schichtweise wachsenden Mikrokapseln angegeben. Ungerade Schichtzahlen entsprechen Chitosansulfat und sind durch negative Zetapotentiale gekennzeichnet. Geradzahlige Schichten entsprechen Chitosan und zeigen positive Zetapotentiale.

### Beispiel 13 Permeabilität von Polyelektrolythüllen (nicht Gegenstand der Erfindung)

Polyelektrolythüllen wurden durch Beschichtung von 3 µm Melaminformaldehydpartikeln unter Verwendung von Natriumpoly(styrolsulfonat) mit einem Molekulargewicht von 70.000 und Poly(allylaminhydrochlorid) mit einem Molekulargewicht von 50.000 erzeugt. Für die konfokale Fluoreszenzmikroskopie wird ein mit Fluoresceinisothiocyanat markiertes PAH (FITC-PAH) eingesetzt (Sukhorukov, Colloids Surfaces A 137 (1998), 253).

Suspensionen von Melaminformaldehydpartikeln mit 3 µm Durchmesser werden mit 13 Schichten PAH und PSS beschichtet. Dann wird durch 5 min Inkubation in 0,1 NaCl der Kern aufgelöst.

Mittels konfokaler Mikroskopie wird die Permeabilität der Polyelektrolytmikrokapseln untersucht. Hierzu wird zunächst eine Lösung von PAH-FITC (Molekulargewicht 50.000) mit den Hüllen auf eine Endkonzentration von 0,5 mg/ml vermischt. Die Permeabilität der Hüllwände für hochmolekulares PAH-FITC ist so gering, daß nach 20 min Inkubation keine Fluoreszenz im Inneren der Kapseln nachweisbar war. Im Gegensatz dazu kann 6-Carbofluoreszein (6-CF) die Wände der Hüllen ohne weiteres durchdringen.

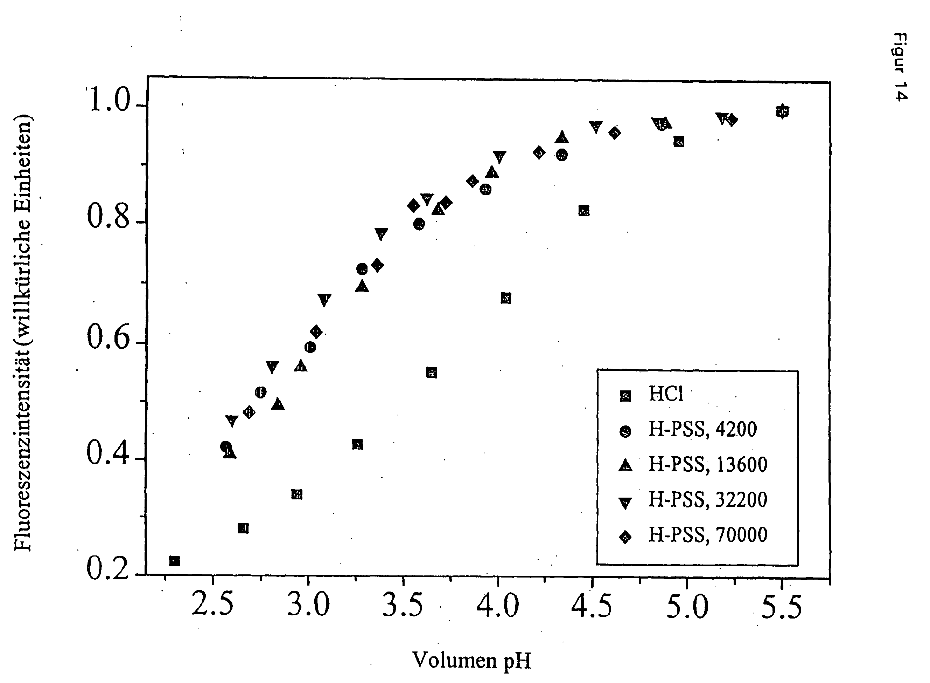

Figur 14 zeigt die Fluoreszenzintensität einer Suspension von 6-CF als Fluoreszenzmarker enthaltenden Polyelektrolytkapseln gegenüber den durch Polystyrolsulfonsäure und HCl titrierten pH-Wert im umgebenden Medium. Die Ergebnisse zeigen, daß Polystyrolsulfonsäure in den jeweils verwendeten Molekulargewichten (70.000-4.200) die Polyelektrolythüllen nicht durchdringen konnte.

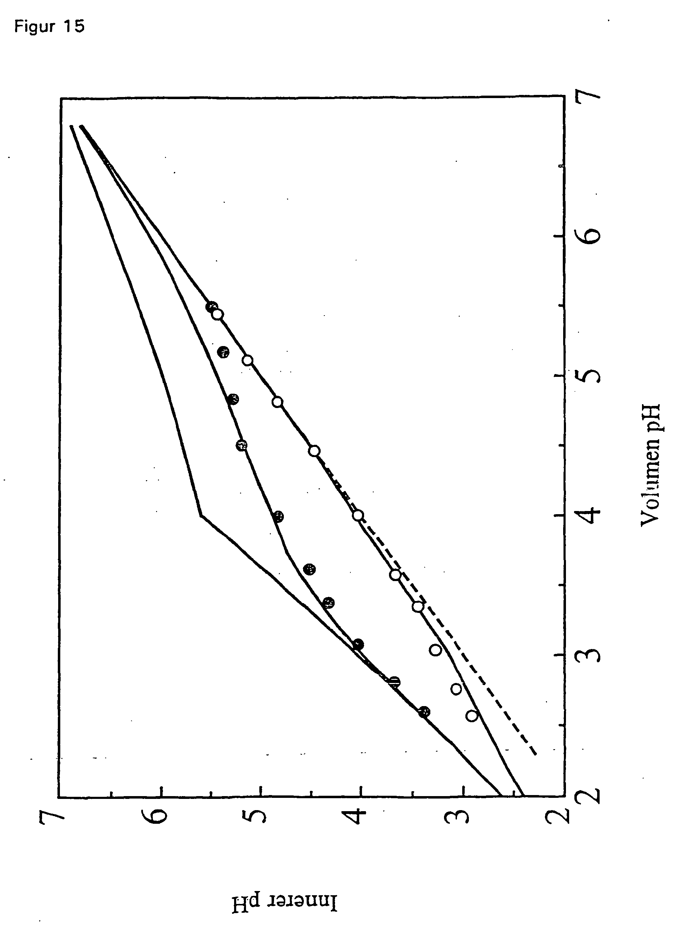

Figur 15 zeigt den pH-Wert im Inneren der Kapseln als Funktion des mit H-PSS (Molekulargewicht 4200) titrierten Volumen pH-Werts in Abwesenheit von Salz und in Gegenwart von 1 mM NaCl. Es ist zu erkennen, daß mindestens innerhalb einer Stunde keine signifikante Diffusion von PSS Polyanionen mit dem jeweils getesteten Molekulargewichten ins Innere der Kapsel stattfindet. Dies führt zum Entstehen eines pH-Gradienten zwischen dem Inneren der Kapsel und dem Volumen. Der pH-Wert im Inneren ist um etwa eine pH-Einheit basischer als der pH-Wert des Volumens. Dies gilt insbesondere für einen Volumen pH-Wert geringer als 5,5.

## Patentansprüche

1. Verfahren zum Aufbringen mehrerer Schichten von
Beschichtungssubstanzen auf Templatpartikel, umfassend die Schritte:
(a) Inkontaktbringen des Templatpartikels mit einer ersten Beschichtungssubstanz in einem fluiden Reaktionsmedium in einem Reaktionsraum, der auf mindestens einer Seite durch eine Filtrationsmembran begrenzt ist, unter Bedingungen, bei denen eine Schicht der ersten Beschichtungssubstanz auf dem Templatpartikel gebildet wird,
(b) Ableiten von mindestens einem Teil des Reaktionsmediums mit gegebenenfalls darin enthaltener überschüssiger erster Beschichtungssubstanz durch die Filtrationsmembran in einen Filtratraum,
(c) Inkontaktbringen des Templatpartikels mit einer zweiten Beschichtungssubstanz in einem fluiden Reaktionsmedium in einem Reaktionsraum, der auf mindestens einer Seite durch eine Filtrationsmembran begrenzt ist, unter Bedingungen, bei denen eine Schicht der zweiten Beschichtungssubstanz auf dem Templatpartikel gebildet wird,
(d) Ableiten von mindestens einem Teil des Reaktionsmediums mit gegebenenfalls darin enthaltener überschüssiger zweiter Beschichtungssubstanz durch Filtrationsmembran in einen Filtratraum, und
(e) gegebenenfalls mehrmaliges Wiederholen der Schritte (a) und (b) oder/und (c) und (d).

2. Verfahren nach Anspruch 1,
**dadurch gekennzeichnet,**
**dass** die ersten und zweiten Beschichtungssubstanzen aus entgegengesetzt geladenen Polyelektrolytspezies oder Gemischen von Polyelektrolytspezies ausgewählt werden.

3. Verfahren nach Anspruch 1 oder 2,
**dadurch gekennzeichnet,**
**dass** die Templatpartikel aus Partikeln mit einem Durchmesser von bis zu 50 µm, insbesondere bis zu 10 µm ausgewählt werden.

4. Verfahren nach einem der Ansprüche 1 bis 3,
**dadurch gekennzeichnet,**
**dass** lösliche Templatpartikel verwendet werden und dass die Auflösung der Kerne nach Beendigung des Beschichtungsvorgangs durchgeführt wird.

5. Verfahren nach Anpsruch 3 oder 4,
**dadurch gekennzeichnet,**
**dass** die Partikel ausgewählt werden aus biologischen Partikeln oder Aggregaten.

6. Verfahren nach einem der Ansprüche 1 bis 5,
**dadurch gekennzeichnet,**
**dass** während oder/und nach Schritt (b) oder/und Schritt (d) ein Waschmedium zugegeben wird.

7. Verfahren nach Anspruch 6,
**dadurch gekennzeichnet,**
**dass** die Zugabe des Waschmediums so erfolgt, dass das Volumen des Mediums in Schritt (b) oder/und Schritt (d) im Wesentlichen konstant bleibt.

8. Verfahren nach Anspruch 1 bis 7,
**dadurch gekennzeichnet,**
**dass** die Filtration im Wesentlichen ohne Druckdifferenz zwischen Reaktionsraum und Filtratraum durchgeführt wird.

9. Verfahren nach einem der Ansprüche 1 bis 8,
**dadurch gekennzeichnet,**
**dass** der Reaktionsraum zumindest während der Schritte (a) oder/und (c) gerührt wird.

10. Verfahren nach Anspruch 9,
**dadurch gekennzeichnet,**
**dass** der Reaktionsraum während des gesamten Prozesses gerührt wird.

## Claims

1. Method for applying a plurality of layers of coating substances to template particles, comprising the steps:
(a) contacting the template particle with a first coating substance in a fluid reaction medium in a reaction chamber which is limited on at least one side by a filtration membrane, under conditions with which a layer of the first coating substance is formed on the template particle,
(b) draining at least part of the reaction medium with, where appropriate, excess first coating substance present therein through the filtration membrane into a filtrate chamber,
(c) contacting the template particle with a second coating substance in a fluid reaction medium in a reaction chamber which is limited on at least one side by a filtration membrane, under conditions with which a layer of the second coating substance is formed on the template particle,
(d) draining at least part of the reaction medium with, where appropriate, excess second coating substance present therein through the filtration membrane into a filtrate chamber, and
(e) where appropriate repeating steps (a) and (b) or/and (c) and (d) a plurality of times.

2. Method according to Claim 1, **characterized in that** the first and second coating substances are selected from oppositely charged polyelectrolyte species or mixtures of polyelectrolyte species.

3. Method according to Claim 1 or 2, **characterized in that** the template particles are selected from particles having a diameter of up to 50 µm, in particular up to 10 µm.

4. Method according to any of Claims 1 to 3,
**characterized in that** soluble template particles are used and **in that** the dissolving of the cores is carried out after completion of the coating process.

5. Method according to Claim 3 or 4, **characterized in that** the particles are selected from biological particles or aggregates.

6. Method according to any of Claims 1 to 5,
**characterized in that** a washing medium is added during or/and after step (b) or/and step (d).

7. Method according to Claim 6, **characterized in that** the addition of the washing medium takes place so that the volume of the medium remains essentially constant in step (b) or/and step (d).

8. Method according to Claims 1 to 7, **characterized in that** the filtration is carried out essentially without a pressure difference between reaction chamber and filtrate chamber.

9. Method according to any of Claims 1 to 8,
**characterized in that** the reaction chamber is stirred at least during steps (a) or/and (c).

10. Method according to Claim 9, **characterized in that** the reaction chamber is stirred throughout the process.

## Revendications

1. Procédé pour appliquer plusieurs couches de substances de revêtement sur des particules matrices, comprenant les étapes de
(a) mise en contact de la particule matrice avec une première substance de revêtement dans un milieu de réaction fluide dans une zone de réaction qui est limitée au moins d'un côté par une membrane de filtration, dans des conditions dans lesquelles il se forme une couche de la première substance de revêtement sur la particule matrice,
(b) séparation d'au moins une partie du milieu de réaction, avec éventuellement la première substance de revêtement en excès qui y est contenue, à travers la membrane de filtration dans une zone de filtrat,
(c) mise en contact de la particule matrice avec une deuxième substance de revêtement dans un milieu de réaction fluide dans une zone de réaction qui est limitée au moins d'un côté par une membrane de filtration, dans des conditions dans lesquelles il se forme une couche de la deuxième substance de revêtement sur la particule matrice,
(d) séparation d'au moins une partie du milieu de réaction, avec éventuellement la deuxième substance de revêtement en excès qui y est contenue, à travers la membrane de filtration dans une zone de filtrat,
(e) éventuellement répétition à plusieurs reprises des étapes (a) et (b) et/ou (c) et (d).

2. Procédé selon la revendication 1, **caractérisé en ce que** les première et deuxième substances de revêtement sont choisies parmi des espèces de polyélectrolytes, ou des mélanges d'espèces de polyélectrolytes, de charge opposée.

3. Procédé selon la revendication 1 ou 2, **caractérisé en ce que** les particules matrices sont choisies parmi des particules ayant un diamètre d'au plus 50 µm, en particulier d'au plus 10 µm.

4. Procédé selon l'une des revendications 1 à 3, **caractérisé en ce que** l'on utilise des particules matrices solubles et **en ce que** l'on effectue une dissolution des noyaux après la fin de l'opération de revêtement.

5. Procédé selon la revendication 3 ou 4, **caractérisé en ce que** les particules sont choisies parmi des particules ou des agrégats biologiques.

6. Procédé selon l'une des revendications 1 à 5, **caractérisé en ce que,** pendant et/ou après l'étape (b) et/ou l'étape (d), on ajoute un milieu de lavage.

7. Procédé selon la revendication 6, **caractérisé en ce que** l'addition du milieu de lavage s'effectue de manière que le volume du milieu dans l'étape (b) et/ou l'étape (d) reste essentiellement constant.

8. Procédé selon l'une des revendications 1 à 7, **caractérisé en ce que** la filtration s'effectue essentiellement sans différence de pression entre la zone de réaction et la zone de filtrat.

9. Procédé selon l'une des revendications 1 à 8, **caractérisé en ce que** la zone de réaction est agitée au moins pendant les étapes (a) et/ou (c).

10. Procédé selon la revendication 9, **caractérisé en ce que** la zone de réaction est agitée pendant toute l'opération.
